(19) **Europäisches Patentamt**

**European Patent Office**

**Office européen des brevets**

(11) **EP 1 642 591 A1**

(12) **EUROPEAN PATENT APPLICATION**
published in accordance with Art. 158(3) EPC

(43) Date of publication:
**05.04.2006 Bulletin 2006/14**

(51) Int Cl.:
*A61K 45/00* (1985.01)   *A61K 48/00* (1990.01)
*A61K 31/7088* (2000.01)   *A61K 9/08* (1974.07)
*A61K 9/12* (1974.07)   *A61K 9/127* (1990.01)
*A61K 9/14* (1974.07)   *A61K 9/72* (1974.07)
*A61K 47/10* (1990.01)   *A61K 47/26* (1990.01)
*A61K 47/30* (1990.01)   *A61K 35/76* (1985.01)
*A61P 11/00* (2000.01)   *A61P 11/02* (2000.01)
*A61P 11/06* (2000.01)

(21) Application number: **04747306.1**

(22) Date of filing: **09.07.2004**

(86) International application number:
**PCT/JP2004/009838**

(87) International publication number:
**WO 2005/004914 (20.01.2005 Gazette 2005/03)**

(84) Designated Contracting States:
**AT BE BG CH CY CZ DE DK EE ES FI FR GB GR HU IE IT LI LU MC NL PL PT RO SE SI SK TR**

(30) Priority: **09.07.2003 WOPCT/JP03/08740**

(71) Applicant: **AnGes MG, Inc.**
**Toyonaka-shi,**
**Osaka 560-0082 (JP)**

(72) Inventors:
• **MORISHITA, Ryuichi,**
**c/o AnGes MG, Inc.**
**Toyonaka-shi,**
**Osaka 560-0082 (JP)**
• **AOKI, Motokuni,**
**c/o AnGes MG, Inc.**
**Toyonaka-shi,**
**Osaka 560-0082 (JP)**
• **OGIHARA, Toshio,**
**c/o AnGes MG, Inc.**
**Toyonaka-shi,**
**Osaka 560-0082 (JP)**

• **KAWASAKI, Tomio,**
**c/o AnGes MG, Inc.**
**Toyonaka-shi,**
**Osaka 560-0082 (JP)**
• **MAKINO, Hirofumi,**
**c/o AnGes MG, Inc.,**
**Toyonaka-shi,**
**Osaka 560-0082 (JP)**
• **SHISHIKURA, Takashi,**
**c/o Meiji Seika Kaisha, Ltd.**
**Yokohama-shi,**
**Kanagawa 222-8567 (JP)**
• **KOYANAGI, Akihiro,**
**c/o Meiji Seika Kaisha, Ltd.**
**Yokohama-shi,**
**Kanagawa 222-8567 (JP)**

(74) Representative: **Bates, Philip Ian**
**Reddie & Grose**
**16 Theobalds Road**
**London WC1X 8PL (GB)**

(54) **MEDICINAL COMPOSITION CONTAINING NF-kAPPA B DECOY FOR TREATING AND PREVENTING RESPIRATORY DISEASES AND METHOD OF USING THE SAME**

(57)    A pharmaceutical composition for treating and preventing diseases, disorders and/or conditions of airway inflammation, airway stenosis or nasal cavity inflammation caused by the expression of a gene regulated by NF-kappaB which contains an NF-kappaB decoy and a pharmaceutically acceptable carrier. The above diseases may be asthma, COPD or rhinitis. Moreover, the above diseases may be diseases caused by an eosinophil abnormality (for example, asthma, rhinitis and COPD). The pharmaceutically acceptable carrier may be a hydrophilic polymer, a liposome and so on.

EP 1 642 591 A1

SEQUENCE LISTING

<110>  AnGes MG, Inc.
       MORISHITA, Ryuichi
       AOKI, Motokuni
       OGIHARA, Toshio
       KAWASAKI, Tomio
       MAKINO, Hirofumi
       SHISHIKURA, Takashi
       KOYANAGI, Akihiro

<120>  NF-kappa B decoy-containing pharmaceutical composition for
       treatment and prevention of respiratory diseases and the use
       thereof

<130>  AN011-1PCT

<150>  PCT/JP03/08740
<151>  2003-07-09

<160>  3

<170>  PatentIn version 3.2

<210>  1
<211>  20
<212>  DNA
<213>  Artificial

<220>
<223>  decoy

<400>  1
ccttgaaggg atttccctcc                                                   20


<210>  2
<211>  20
<212>  DNA
<213>  Artificial

<220>
<223>  decoy

<400>  2
ttgccgtacc tgacttagcc                                                   20


<210>  3
<211>  10
<212>  DNA
<213>  Artificial

<220>
<223>  decoy

<400>  3
gggrhtyyhc                                                              10

**Description**

FIELD OF THE INVENTION

The present invention relates to a composition comprising a compound (e.g., a nucleic acid and a homolog thereof) which specifically binds to a site on a chromosome, to which site a transcriptional regulatory factor binds, in particular **NF-κB**, and a method of using the same. More particularly, the present invention relates to a composition comprising an NF-κB decoy compound and a method of using the same.

In particular, the present invention provides a composition for treating, improving or preventing airway inflammation, airway stenosis or nasal cavity inflammation, and a method of use thereof with significant effects, specifically, for example, a composition for radically treating, improving and preventing asthma or nasal inflammation.

BACKGROUND ART

In March, 1993, the WHO (World Health Organization) reported that "it is a fact that morbidity of asthma is increasing globally" and published findings that amongst the world population of 5.5 billion, asthma patients have exceeded a total of one hundred million ("International guidance towards asthma control - global strategy for asthma control and prevention, NHLBI/WHO Workshop Report" ed. Sohei MAKINO, Kokusai Igaku Shuppan (International Medical Publishers, Inc.) 1995). In particular, the morbidity of asthma in infants is increasing.

In Japan, a trend of increased infantile asthma is evident, and according to a school health research by Mombusho (Ministry of Education), the morbidity of infantile asthma peaked in 1994, and has increased by between two to four times compared to the period between 1975-1984.

According to statistics published by Koseisho (Ministry of Health and Welfare) as of October, 1993, a total of 1,066,000 people in Japan has asthma (male: 586,000; female: 480,000), and thus it is calculated that 0.9 percent of the population is affected by asthma.

Further, depending on the method of research there are between 2 to 3 million asthma patients in Japan, (about 2-3 % of the population), and the number of deaths ascribed to asthma each year is estimated to be about 6,000-7,000.

As such, the present trend both in Japan and elsewhere, is for increased asthma-related morbidity. Further, the mortality associated with asthma has not decreased; the risk of death due to asthma is high, particularly in the case of intractable asthma.

Inflammation of the airway mucous membranes play an important role in the pathology of bronchial asthma.

That is, once the airway mucous membrane receives an allergen stimulus, inflammatory cells such as mast cells or eosinophils produce a variety of cytokines, histotoxic proteins, and the like. At that point, inflammatory stimulation of cells causes the activation of transcription factors such as NF-κB and AP-1 which are activated by inflammatory stimuli to enhance the expression of a variety of genes, such as a variety of cytokines.

Inflammatory factors such as cytokines are responsible for mediating interaction between immune-system cells, and play an important role in the immune response to allergens or microorganisms.

However, an excessive allergic response or inflammation, mediated via these factors results in disadvantageous phenomena such as asthma attacks in patients having bronchial asthma, airway stenosis and the like.

Mast cells and eosinophils carry receptors for cytokines on the surface-membrane thereof. Once cytokines produced by other cell or by itself are bound to such receptors, the signal is transmitted to the nucleus via intracellular signaling to promote the transcription of a variety of genes, thereby producing new cytokines, chemokines and cellular adhesion molecules, based on the transcripts.

In this case, transcription factors play an important role in regulating the transcriptional activity of genetic information in the nucleus. Important transcription factors in airway inflammation include NF-κB, AP-1, NF-AT (nuclear factor of activated T cells), and in particular, NF-κB plays a key role.

Factors produced in response to NF-κB promotion of transcription include a wide variety of factors such as inflammatory cytokines, chemokines, cytotoxic proteins, cellular adhesion molecules, cell membrane receptors related to the immune system, and the like.

To date, it has been reported that in human mast cells (MC), IgE dependent NF-κB is activated by inflammatory antibody stimulus, and TNF-alpha (tumor necrosis factor alpha (α)) is recognized to promote NF-kB activation. Further, it is known that GM-CSF (Granulocyte Macrophage Colony Stimulating Factor) is important for NF-κB activation in eosinophils (J. Immunol.,169(9), 5287-93, 2002.).

TNF-α present in the airway has a biological action similar to IL-1, and is related to airway inflammation and the hyperreaction in the airway. Further, adhesion molecules expressed in the airway epithelium and endothelium activated by IL-1 or IL-4, in particular, VCAM-1 results in the infiltration of eosinophils into the airway, and is involved in hyperreaction in the airway. In animal tests, administration of antibodies against ICAM-1 suppresses the infiltration of eosinophils and reactivity in the airway.

Conventional treatment for asthma is mainly directed to the control of inflammation, and is classified into the following: long-term controlling agents (controller) continuously used for long-term control, and attack treatment agents (reliever) used for the short-term treatment of asthma attacks.

Specifically, inhaled steroids, anti-allergic agents, sustained-release theophyllines, and the like, have been used as controller agents, and inhaled β2 receptor activators, aminophylline, oral or i.v. steroids, and the like, have been used as reliever agents.

However, notwithstanding the attack relieving action of reliever agents, treatment using conventional controller agents has not attained sufficient effects as, as mentioned above, asthma-related morbidityis increasing and asthma-related mortality has not reduced.

Therefore, research into the treatment of asthma has been recently shifting from such conventional symptomatic treatment to suppression of airway inflammation.

A representative transcriptional factor, NF-κB is a transcriptional regulatory factor consisting of a heterodimer of p65 and p50. NF-κB is typically localized in the cytoplasm where it is bound by its inhibitory factor IκB so that nuclear translocation of NF-κB is prevented. However, when a stimulus, such as cytokines, ischemia, reperfusion, or the like, is applied, IκB is degraded by phosphorylation. As a result, NF-κB is activated and translocates into the nucleus. In the nucleus, NF-κB binds to an NF-κB binding site on a chromosome and promotes the transcription of a gene downstream thereof. A number of genes located downstream of NF-κB binding sites, such as inflammatory cytokines (e.g., IL-1, IL-6, IL-8, tumor necrosis factor $\alpha$ (TNF $\alpha$), etc.) and adhesion molecules (e.g., (e.g., VCAM-1, ICAM-1, etc.) are known.

Regardless of the existence of a number of patients, bronchial asthma, allergic asthma, infantile asthma and the like are the diseases which are difficult to treat radically, and such radical treatment has been awaited for long period of time. Present treatment is largely based upon symptomatic treatments and is mainly directed to the administration of bronchodilators such as theophylline, and the like. However, such symptomatic treatments can only be initiated after attack has been triggered, and thus complete suppression of patient's symptoms is impossible, and radical treatment is not possible. Accordingly, there is long felt demand for therapies which provide radical treatment.

Rhinitis has a number of symptoms, in addition to symptoms common for the common cold such as nasal congestion, nasal discharge, sneezing and the like, a variety of symptoms such as nasal hemorrhage, impaired sense of smell and taste, headache, lack of concentration, and the like.

Rhinitis is classified, based on the cause/causative substance or clinical symptoms thereof, into the following categories: allergic rhinitis, pollinosis, acute rhinitis, chronic rhinitis, hypertrophic rhinitis, chronic sinusitis (empyema) and deflected septum.

Allergic rhinitis, a representative rhinitis, is caused by the inhalation of inhaled antigens such as dust, dust mites, pollen, and the like, to induce nasal symptoms such as sneezing, nasal discharge and nasal congestion, and the like. At first, there is no symptoms when an antigenic substance is inhaled. However, after such an antigen is initially inhaled, the number of an antibody thereto in the body increases gradually, and when the antibody titer exceeds a certain level, and such an antigenic substance is inhaled, then symptoms occur.

The pathology thereof is basically believed to be an allergic inflammation (Rhinology, 41(2), 80-6, 2003.).

The number of patient with pollenosis due to *sugi* (Cryptomeria japonica), has been increasing in Japan, and now is recognized to have reached 15 million. In particular, in Japanese metropolitan areas, it is said that one fifth of the population has pollenosis. The reasons for this are considered to be as follows:

(1) Increasing amount of *sugi* pollen scatter;
(2) Air pollution by exhaust gas from diesel engines facilitates the production of IgE antibodies.
(3) The increase of stress facilitates allergic reaction.
(4) Promotion of production of IgE antibody due to Westernized dietary habits.
(5) Reduction of parasite infection.

As used herein the term "allergic rhinitis" refers to a rhinitis causing a reaction of the nasal cavity mucosa against a specific substance, and is possibly associated with sneezing, nasal discharge and nasal congestion, which are the three major symptoms, and possible further symptoms such as ocular pruritus, ocular hyperemia, and systemic symptoms such as lack of appetite and fatigue.

Substances causing allergy (allergens) include pollen *(sugi, hinoki* (Chamaecyparis obtusa) and the like), ragweed, dust mites, house dust, pet hair, and the like, and these are divided into seasonal allergens (such as *sugi* pollen in spring) and year-round allergens.

A variety of methods for treating rhinitis have been proposed. However, to date, only removal of allergens or symptomatic therapies are available, and many of such symptomatic therapies have side effects.

Further, chronic obstructive pulmonary disease (COPD) is ranked at the fourth largest cause of death cause globally. However, there are a number of patients who do not recognize it before it has become severe, and thus it has become one of the most severe health problems of the 21 century. In the United States, the mortality of COPD increases with aging and is ranked as the fourth or fifth greatest cause of death at ages of 45 or greater, depending on the age stratification. During the period between 1965 through to 1998, although the mortality of the other diseases has reduced, the mortality of COPD has strikingly increased, and there is strong demand for radical solution therefor.

The number of patients affected with COPD is increasing in recent years, and thus is expected to further increase in the future. As present, there is no radical treatment for COPD, and medical expense by social assurance associated with COPD is also large. COPD is characterized as a pathogenic condition of irreversible limitation of airflow. Airflow limitation is usually progressive, and differs in the extent thereof depending on the amount or types of toxic gas inhaled. Airflow limitation is caused by disruption of lung parenchyma caused by inflammatory response in the peripheral airway in response to the gas inhaled. WHO and World Bank conduced research in 1990, which reported that the global morbidity thereof is 9.34 per 1000 population (male) and 7.33 per 1000 population (female). At present COPD is increasing, and was ranked at the sixth leading cause of death in 1990, and is expected to be ranked up to the third by 2020. In Japan, at present, there are about 50,000 patients who are now receiving oxygen therapy at home due to this disease. The number of patients was 220,000 according to statistics published by the Ministry of Health and Welfare in 1996. However, according to the statistics published by the Lung disease epidemology research association (haishikkan ekigaku chosa kenkyukai), the estimated number of COPD patients is 5,300,000 in Japan. Further, the morbidity of COPD of the age of 40 or greater is 8.5 % which is comparable to that of the global morbidity. To date, it has been believed that there are few patients in Japan, however, there are a number of patients that have not yet been diagnosed and therefore in view of the actual number of patients, there is increasing demand for the radical treatment of COPD.

COPD is a disease in which the "airway", which is the passageway for air during respiration, receives damage and thus gradually lowers respiratory function. Diseases that were previously called "lung emphysema" and "chronic bronchitis" are now collectively called COPD.

COPD is initiated with normal symptoms, and gradually progresses, and thus is believe to be "lung lifestyle-related disease", whereby when a patient is diagnosed, he/she is already in a severe condition. COPD is a disease where one's movement is restricted due to the difficulty of breathing in a severe condition, and systemic disorders will occur, which is a disease with an extreme severity. Therefore, in terms of improvement of symptoms, there is a large demand for therapy therefor.

Therapies for COPD at present only include symptomatic therapies and improvement of lifestyle, which are passive methods, and thus there are no positive methods or radical treatments. For example, it is proposed to quit lifestyle habits with high risk of COPD, such as smoking. In addition, drug therapy to facilitate respiration, rehabilitation, oxygen therapy and the like, which are symptomatic therapies are also practiced. Vaccination for prevention of bronchial infections and therapy for complications and the like are also practiced. For the most severe patients, surgical treatment is also considered when all the conditions are satisfied. However, it cannot be said that such surgical treatment improves symptoms satisfactorily.

As such, there is no effective treatment for COPD at present, and there is no means to delay the progress of the disease at present. As described above, to date, the therapies practiced today are only symptomatic therapies such as oxygen inhalation, exterior positive ventilation, and the like. As described above, when necessary, surgical resection of diseased lung tissue is performed. However, there are a number of reports that the above treatments do not provide improvement of long-term prognosis of COPD. Moreover, the effects of administering agents such as bronchodilators, expectorants, and the like, are limited.

Patent Reference 1 (WO 03/105780 pamphlet) and Patent Reference 2 (US 6303582) disclose technology for producing microparticles of a nucleic acid. However, the references fail to describe whether or not small nucleic acids, such as a decoy, can be produced as microparticles whilst still functioning against inflammatory diseases.

It is an object of the invention to provide a composition and a use thereof adapted for treating a disease of the respiratory system caused by the expression of a gene regulated by NF-κB, such as a disease, disorder and/or condition including airway inflammatory disease, airway stenosis or nasal cavity inflammatory disease.

SUMMARY OF INVENTION

The present invention provides a composition comprising NF-κB decoy as a main ingredient- for treating and/or preventing a disease, disorder and/or condition of respiratory system due to expression of a gene regulated by NF-κB, and a method of using the composition for treating and/or preventing the disease.

The present inventors have discovered that the administration of an NF-kB decoy is effective in treating and/or preventing a disease, disorder and/or a condition of the respiratory system due to expression of a gene regulated by NF-κB, or due to an abnormality of eosinophils and/or neutrophils, to achieve the present invention.

The present invention is related to a pharmaceutical composition for treating and preventing a disease, disorder and/or condition due to the expression of a gene regulated by NF-κB The composition comprises an NF-κB decoy, a derivative, variant or fragment thereof and a pharmaceutically acceptable carrier, and the derivative, variant or fragment is a decoy having a biological activity.

The present invention is also related to a pharmaceutical composition for treating and/or preventing a disease, disorder and/or condition of the respiratory system related to an eosinophil abnormality, comprising an NF-κB decoy and a pharmaceutically acceptable carrier.

Preferably, the disease is COPD, asthma or rhinitis.

Preferably, the disease is a disease related to an eosinophil abnormality. Such a disease related to the an eosinophil abnormality includes, for example, an asthma such as bronchial asthma, infantile asthma, allergic asthma, atopic asthma, steroid refractory asthma (SRA), non-allergic asthma (endogenous asthma, exogenous asthma such as aspirin asthma, cardiac asthma and infectious asthma); and other eosinophil abnormalities such as rhinitis, allergic diseases,and the like.

Preferably, said pharmaceutically acceptable carrier is a hydrophilic polymer, a nonsoluble liposome, a carbohydrate or a nonsoluble additive.

Preferably, said pharmaceutically acceptable carrier is one or more selected from the group consisting of a liposome, lactose, trehalose, sucrose, mannitol and xylitol.

Preferably, said NF-κB decoy is a decoy set forth in SEQ. ID NO: 1.

Preferably, said disease, disorder and/or condition of respiratory system is airway inflammatory disease, airway stenosis or nasal cavity inflammatory disease.

Preferably, said disease, disorder and/or condition of respiratory system is COPD, asthma or rhinitis.

Preferably, said disease, disorder and/or condition of respiratory system is COPD.

Preferably, said disease, disorder and/or condition of respiratory system is asthma.

Preferably, said disease, disorder and/or condition of respiratory system is rhinitis.

Preferably, said disease, disorder and/or condition of respiratory system is an asthma selected from the group consisting of bronchial asthma, infantile asthma, allergic asthma, atopic asthma, steroid refractory asthma, non-allergic asthma, endogenous asthma, exogenous asthma, aspirin asthma, cardiac asthma and infectious asthma; or a rhinitis selected from the group consisting of allergic rhinitis, pollinosis, acute rhinitis, chronic rhinitis, hypertrophic rhinitis, chronic sinusitis (emphysema) and deflected septum.

In another aspect, the present invention is related to the use of an NF-κB decoy for the manufacture of a medicament or pharmaceutical composition for treating and/or preventing a disease, disorder and/or condition of the respiratory system due to the expression of a gene regulated by NF-κB, or a disease, disorder and/or condition of the respiratory system due to an eosinophil abnormality.

In another aspect, the present invention comprises the above-mentioned composition of the present invention, which is adapted for administration to the respiratory system.

Preferably, the administration to the respiratory system comprises the intraairway administration or transairway absorption.

Preferably, the administration to the respiratory system is characterized by the administration to the airway by atomization or inspiration

Preferably, the administration into the respiratory system comprises administration by metered dose inhaler (MDI), dry powder inhaler (DPI) or nebulizer.

Preferably, the composition is provided as a dry powder.

Preferably, said dry powder is a microparticle produced by means of one of selected from the group consisting of a bowl mill, a bead mill, a jet mill, an ultimizer, a mortar, a stonemill, spray drying and supercritical fluid.

Preferably, the dry powder has an aerodynamic average particle size of about 0.01 to about 50 micrometer.

Preferably, the dry powder has an aerodynamic average particle size of about 0.05 to about 30 micrometer.

Preferably, the dry powder has an aerodynamic average particle size of about 0.1 to about 10 micrometer.

Preferably, a dosage of 10 μg to 100 mg per round is provided in the present formulation.

Preferably, a dosage of 50 μg to 50 mg per round is provided in the present formulation.

Preferably, a dosage of 10 mg or less per round is provided in the present formulation.

Preferably, the administration to the respiratory system comprises nasal absorption.

Preferably, the formulation is a formulation selected from the group consisting of a nasal drop, a nasal spray agent, an agent for a nebulizer, an agent for respirator and a powder administration formulation.

Preferably, the formulation is a nasal drop for rhinitis.

Preferably, the present invention is characterized in that the NF-κB decoy is encapsulated in an HVJ-E envelope vector.

Preferably, the administration to the respiratory system comprises administration to the lung.

In another aspect, the present invention provides a device for treating the respiratory system comprising a composition according to the present invention, and means for administrating the composition to the respiratory system.

Preferably, the means for administration to the respiratory system comprises means selected from the group consisting of means for administration to the lung, means for transairway administration, means for transairway absorption and means for nasal absorption.

Preferably, said means for administration into the airway comprises a metered dose inhaler (MDI), dry powder inhaler (DPI) or a nebulizer.

In another aspect, the present invention provides a method for manufacturing a particle comprising NF-κB decoy, comprising the steps of: A) providing the decoy and a pharmaceutically acceptable carrier; B) drying and heating the decoy and the carrier to a temperature sufficient for particulization; and C) obtaining particles having a desired particle size.

Preferably, the temperature is 50 degrees Celcius or lower.

Preferably, the temperature is 100 degrees Celcius or lower.

Preferably, the dry powder has an aerodynamic average particle size of about 0.01 to about 50 micrometer.

Preferably, the dry powder has an aerodynamic average particle size of about 0.05 to about 30 micrometer.

Preferably, the dry powder has an aerodynamic average particle size of about 0.1 to about 10 micrometer.

In another aspect, the present invention provides a method for treating and/or preventing a disease, disorder and/or condition of the respiratory system due to expression of a gene regulated by NF-κB, comprising the step of: A) administration of a composition comprising an NF-κB decoy and a pharmaceutically acceptable carrier to the respiratory system of a subject.

Preferably, said disease, disorder and/or condition of the respiratory system is airway inflammatory disease, airway stenosis or nasal cavity inflammatory disease.

Preferably, said disease, disorder and/or condition of the respiratory system is COPD, asthma or rhinitis.

Preferably, said administration to the respiratory system comprises administration into the airway or transairway absorption.

Preferably, said administration to the respiratory system is administration to the airway by atomization or inspiration.

Preferably, said administration into the airway comprises administration by metered dose inhaler (MDI), dry powder inhaler (DPI) or nebulizer.

Preferably, the administration is achieved by means selected from the group consisting of a nasal drop, a nasal spray, a nebulizer, a respirator and powder administration.

In another aspect, the present invention provides a method for treating and/or preventing a disease, disorder and/or condition of respiratory system due to an eosinophil abnormality, comprising the step of: A) administration of a composition comprising an NF-κB decoy and a pharmaceutically acceptable carrier to the respiratory system of a subject.

In another aspect, the present invention is related to the use of an NF-κB decoy for the manufacture of a medicament for treating and/or preventing a disease, disorder and/or condition of the respiratory system due to expression of a gene regulated by NF-κB.

In another aspect, the present invention is related to the use of an NF-κB decoy for the manufacture of a medicament for treating and/or preventing a disease, disorder and/or condition of respiratory system due to an eosinophil abnormality.

EFFECTS OF THE INVENTION

The present invention allows treatment, such as therapy or prevention, of respiratory diseases such as airway inflammatory disease, airway stenosis or nasal cavity inflammatory disease, and the like.

BRIEF DESCRIPTION OF THE INVENTION

Figure **1** is an example of experimental protocols showing the effects of the pharmaceutical composition according to the present invention in the case of asthma model (Figure 1A). Figure 1B and 1C show the effects of inhalation of the decoy.

Figure **2** depicts a result showing the effects of a pharmaceutical composition according to the present invention in the bronchoalveolar lavage fluid from an animal challenged with ovalbumin. Figures **2A** and **2B** show raw data for calculating numbers of eosinophils. Figure 2C depicts the effects of the present invention, which shows from the left, control, ovalbumin challenge, aerosol treatment (2mg x 2) and liquid treatment (0.5 mg x 1), respectively.

Figure **3** depicts a result showing the effects of a pharmaceutical composition according to the present invention in the bronchoalveolar lavage fluid from an animal challenged with ovalbumin. Figure **3A** (eosinophil cell number) and Figure **3B** (eosinophil percentage) show the effects of the present invention. Each graph shows, from the left, control, ovalbumin challenge, naked decoy (500 microgram), HVJ (200; comprising HVJ-E 10000 HAU and decoy 200 μg),

HVJ(500; comprising HVJ-E 25000 HAU and decoy 500 $\mu$g) and vector (HVJ-E 25000 HAU only, no decoy). # shows the statistical significance of control vs. ovalbumin challenge.

Figure **4** shows examples of the behavior of eosinophils.

Figure **5** shows results for a powder inhalation agent preparation of NF-$\kappa$B decoy of the present invention.

Figure **6** shows results for a powder inhalation agent preparation comprising lactose, which was used as a control.

Figure **7** depicts the results of NF-$\kappa$B decoy in a rat asthma model (AHR). The legend of the figure is as follows: ##: p<0.01 vs saline group (Student's t-test);*, **: p<0.05, 0.01, respectively, vs control group (Dunnett's multiple comparison test).

Figure **8** depicts the results of NF-$\kappa$B decoy in a rat asthma model (BALF). The legend of the figure is as follows: ##: p<0.01 vs saline group (Student's t-test);*, ** : p<0.05,0.01, respectively, vs control group (Dunnett's multiple comparison test).

Figure **9** depicts the effect of NF-$\kappa$B decoy in a rat rhinitis model. The legend of the figure is as follows: ##: p<0.01 vs saline group (Student's t-test);*, ** : p<0.05,0.01, respectively, vs control group (Dunnett's multiple comparison test).

DESCRIPTION OF SEQUENCE LISTING

SEQ ID NO: **1** is an NF-$\kappa$B decoy.

SEQ ID NO: **2** is a scrambled NF-$\kappa$B decoy.

SEQ ID NO: **3** is a variant of an NF-$\kappa$B decoy.

Hereinafter, the present invention will be described by way of preferred embodiments. It will be understood by those skilled in the art that the embodiments of the present invention can be appropriately made or carried out based on the description of the present specification and commonly used techniques that are well known in the art. The function and effect of the present invention can be easily recognized by those skilled in the art.

BEST MODE FOR CARRYING OUT THE INVENTION

Hereinafter, the present invention will be described. It should be understood throughout the present specification that articles for singular forms (e.g., "a", "an", "the", etc. in English; "ein", "der", "das", "die", etc. and their inflections in German; "un", "une", "le", "la", etc. in French; "un", "una", "el", "la", etc. in Spanish, and articles, adjectives, etc. in other languages) include plural referents unless the context clearly dictates otherwise. It should be also understood that the terms as used herein have definitions typically used in the art unless otherwise mentioned. Accordingly, unless otherwise defined, all terminology and technical terms used herein will have the same meanings as those generally understood by those skilled in the art belonging to the filed of the present invention. If there is contradiction, the present specification (including the definition) takes precedence.
The embodiments provided herein below are provided for the purpose of better understanding of the present invention, and thus it should be understood that the scope of the present invention should not be limited to the following description. Accordingly, it should be apparent for those skilled to make any modification within the scope of the present invention in view of the description of the present specification.
The term "decoy" or "decoy compound" refers to a compound which binds to a site on a chromosome, to which NF-$\kappa$B binds to, or a site on a chromosome(hereinafter referred to as a target binding site), to which another transcription regulatory factor for a gene controlled by NF-$\kappa$B binds to, and antagonizes the binding of NF-kB to target binding sites thereof. Representatively, the decoy or the decoy compound includes a nucleic acid and analogs thereof.
When a decoy is present within a nucleus, the decoy conflicts with a transcription regulatory factor competing for a target binding site for the transcription regulatory factor. As a result, a biological function which would be generated by binding of the transcription regulatory factor to the target binding site is inhibited. The decoy contains at least one nucleic acid sequence capable of binding to a target binding sequence. A decoy can be used for preparation of a pharmaceutical composition according to the present invention as long as the decoy can bind to a target binding sequence.

Preferable examples of a decoy include, but are not limited to, an oligonucleotide comprising 5'-CCT-TGA-AGG-GAT-TTC-CCT-CC-3'(SEQ ID NO: 1) (NF-κB decoy), or a complement thereof, a variant thereof, or a compound including one or more of these molecules, and the like. The oligonucleotides may be DNA or RNA and may also include a modified nucleic acid and/or pseudonucleic acid therein. Further, these oligonucleotides may be mutants thereof, or compounds containing them therein. The oligonucleotides may be a single strand or double stranded, or may be linear or annular, or may be branched or linear. A mutant refers to a nucleic acid having a sequence containing mutation, substitution, insertion, or deletion in one of the above-described sequences, and which is capable of specifically antagonizing NF-KB, or another transcription regulatory factor for a gene controlled by NF-κB, with respect to its binding to the binding site thereof. More preferable decoys include double-strand oligonucleotides containing one or a plurality of the above-described nucleic acid sequences, or mutants thereof. Representative variants of NF-κB decoy include, but are not limited to: those having a nucleic acid sequence set forth in SEQ ID NO:3.

The oligonucleotides for use in the present invention include oligonucleotides modified so as to resist in vivo degradation, and the like, such as oligonucleotides (S-oligo) having a thiophosphatediester bond which is a phosphatediester bond whose oxygen atom is replaced with a sulfur atom, oligonucleotides whose phosphatediester bond is substituted with a methylphosphate group having no electronic charge, and the like.

Molecular biological techniques, biochemical techniques, and microbiological techniques as used herein are well known in the art and commonly used, and are described in, for example, Ausubel F.A.et al ed. (1988) Current Protocols in Molecular Biology , Wiley New York , NY; Sambrook J. et al. (1989)Molecular Cloning: A Laboratory Manual,2nd Ed., Cold Spring Harbor Laboratory Press, Cold Spring Harbor, NY, Supplement Experimental Medicine "Gene introduction and expression analysis methods", Yodosha, 1997 and the like.

The terms "nucleic acid", "nucleic acid molecule", "polynucleotide" and "oligonucleotide" as used herein have the same meaning otherwise noted and refer to a polymer consisting of a series of nucleotides. Nucleotide refers to a nucleoside constituting a phosphate ester. Basic portions include nucleotides having pyrimidine base or purine base (pyrimidine nucleotide and purine nucleotide) . Nucleotides constituting a polynucleotide include DNA or RNA and the like. As described above, a decoy usually employs these forms of a nucleic acid.

The sequence obtained by conducting homology search using a software such as BLAST, FASTA and the like based on the sequence of the decoy of the present invention, against genome data as those included in the Human Genome Project, or gene information databases such as GenBank, are also within the scope of the present invention. Sequences obtained by biologically screening a library produced based on such a database (for example, obtaining the same under stringent conditions).

The similarity, identity and homology of base sequences are herein compared using FASTA (sequence analyzing tool) with the default parameters, unless otherwise noted.

As used herein, the term "corresponding" gene or sequence (e.g., a decoy,promoter sequence or the like) refers to a gene in a given species, which has, or is expected to have, a function similar to that of a predetermined gene in a species as a reference for comparison. When there are a plurality of genes having such a function, the term refers to a gene having the same evolutionary origin. Therefore, a gene corresponding to a given gene may be an ortholog of the given gene. Therefore, genes corresponding to those such as murine NF-κB or sequences can be found in other animals such as a human, rat, pig, cattle or the like. Such a corresponding gene can be identified by techniques well known in the art. Therefore, for example, a corresponding gene in a given animal can be found by searching a sequence database of the animal (e.g., human, rat) using sequences such as murine NF-κB for a reference gene as a query sequence. Such corresponding genes can be readily obtained by those skilled in the art using sequence databases or libraries. In the present invention, sequences corresponding to the particular sequence of the present invention (for example, SEQ ID NO: 1) will also be encompassed thereby.

An "oligonucleotide derivative" or a "polynucleotide derivative" includes a nucleotide derivative, or refers to an oligonucleotide or a polynucleotide having linkages between nucleotides different from typical linkages, which are interchangeably used. Examples of such an oligonucleotide specifically include an oligonucleotide derivative in which a phosphodiester bond in an oligonucleotide is converted to a phosphorothioate bond, an oligonucleotide derivative in which a phosphodiester bond in an oligonucleotide is converted to a N3'-P5' phosphoroamidate bond, an oligonucleotide derivative in which a ribose and a phosphodiester bond in an oligonucleotide are converted to a peptide-nucleic acid bond, an oligonucleotide derivative in which uracil in an oligonucleotide is substituted with C-5 propynyl uracil, an oligonucleotide derivative in which uracil in an oligonucleotide is substituted with C-5 thiazole uracil, an oligonucleotide derivative in which cytosine in an oligonucleotide is substituted with C-5 propynyl cytosine, an oligonucleotide derivative in which cytosine in an oligonucleotide is substituted with phenoxazine-modified cytosine, an oligonucleotide derivative in which ribose in DNA is substituted with 2'-O-propyl ribose, and an oligonucleotide derivative in which ribose in an oligonucleotide is substituted with 2'-methoxyethoxy ribose.

As used herein, the term "biological activity" refers to the activity which a certain factor (e.g., a polynucleotide or polypeptide) has within an organism, including activity exhibiting various functions. For example, when the certain factor is a transcriptional factor, its biological activity includes the activity of regulating transcriptional activity. When the certain factor is an enzyme , its biological activity includes enzymatic activity. As another example, when the certain factor is a ligand, its biological activity includes binding to a receptor to which the ligand corresponds. In an embodiment of the present invention, such a biological activity comprises binding activity to at least one transcriptional factor(s). A method for measuring binding activity of such a transcriptional factor, may be achieved by mixing a transcriptional factor and a binding sequence of a transcriptional factor, and determining the resultant complex caused by the binding (by, for example, observation by electrophoresis). Such a method is well known in the art and routinely used.

As used herein the term "nucleotide" may be naturally occurring or non-naturally occurring. "Nucleotide derivative" refers to a derivative which is different from a naturally occurring nucleotide but has a similar function as that of such a nucleotide. Such a nucleotide derivative and nucleotide analog is well known in the art. Examples of such a nucleotide derivative include, for example, but are not limited to phosphorothioate, phosphoramidate, methyl phosphonate, chiral methyl phosphonate, 2-O-methyl ribonucleotide, peptide-nucleic acid (PNA).

As used herein the term "variant" refers to a substance which is altered in a part of the original substance, such as a polynucleotide. Such a variant includes a variant having substitution, addition, or deletion, a truncated variant, an allelic variant, and the like. An allele refers to genetic variants which are located in the same genetic locus as one another and may be differentiated from each other.

The decoy of the present invention can be produced with chemical or biochemical synthesis methods known in the art. For example, when a nucleic acid is used as a decoy compound, nucleic acid synthesis methods commonly used in genetic engineering can be employed. For example, a DNA synthesizer may be used to directly synthesize intended decoy nucleic acids. Further, these nucleic acids, nucleic acids containing the nucleic acids, or parts thereof may be synthesized, followed by amplification using a PCR method, a cloning vector, and the like. Furthermore, nucleic acids obtained by these methods are cleaved using a restriction enzyme, or the like, and linked or the like using DNA ligase, or the like to produce an intended nucleic acid. To obtain decoy nucleic acids which are more stable in cells, base, sugar and phosphate portions of the nucleic acids may be subjected to chemical modification, such as alkylation, acylation, or the like.

The present invention provides a pharmaceutical composition comprising the above-described decoy compound alone or in combination with a stabilizing compound, a diluent, a carrier or another component, or a pharmaceutical agent.

As used herein the term "patient" or "subject" refers to an organism to which the treatment or composition of the present invention is applied. Patients may be any animal such as a primate, a rodent or the like, as long as the treatment or composition of the present invention can be applied thereto. Preferably, the patient may be a human.

As used herein the term "a disease, disorder and/or condition due to expression of a gene regulated by NF-κB" refers to a disease or a disorder for which regulation (for example, augmentation, reduction, delay or the like) of the expression (for example, translation or transcription) by NF-κB, a transcriptional factor, is the cause thereof. Such a disease, disorder or condition includes, but is not limited to: asthma, rhinitis, COPD and the like.

As used herein the term "a disease, disorder and/or condition related to an eosinophil abnormality " refers to any disease, disorder and/or condition where, as a cause or consequence, the eosinophil level in the body (for example in the blood) is outside the normal level. Eosinophils have IgE receptor on the surface, and play an important role in allergic diseases.

Such diseases related to eosinophil abnormality include, but are not limited to: asthma (bronchial asthma, infantile asthma, allergic asthma, atopic asthma, steroid refractory asthma (SRA), non-allergic asthma (endogenous asthma, exogenous asthma such as aspirin asthma, cardiac asthma and infectious asthma; and other eosinophil abnormalities such as rhinitis, allergic diseases and the like.

As used herein the term "a disease, disorder and/or condition related to the respiratory system" refers to any disease, disorder and/or condition related to the respiratory system. Such a disease related to the respiratory system includes, but is not limited to airway inflammatory disease, airway stenosis or nasal cavity inflammatory disease.

As used herein, diseases related to the respiratory system include, COPD, asthma, rhinitis, and the like.

As used herein the term "COPD" (the abbreviation of Chronic Obstructive Pulmonary Disease) collectively refers to a disease in which access of air to the lung chronically deteriorates and thus gradually becomes diseased. It substantially encompasses any disease which has been called "chronic bronchitis" and "pulmonary emphysema" to date. Conditions of COPD include those, for example, in which cough and sputum are continuously recognized every day even if a patient does not catch a cold, and which a patient feels breathlessness when moving his/her body such as ups and downs of staircases, and the like. Some patients will only recognized their breathlessness when they feel fatigue in walking in the same pace as the same generation without cough and sputum. These are usual conditions, and thus are misrecognized to be due to aging. However, the COPD is characterized by continuous expression of cough, sputum and breathlessness and the like.

COPD is mainly divided in to what is conventionally called "chronic bronchitis", in which disease of bronchus and bronchulus, and "pulmonary emphysema" which is characterized in disease of alveoli pulmonis. Most patients are seen with the two types of disease at the same time. Even if a patient has chronic bronchitis, there are cases where cough and sputum are not always associated, and therefore it has recently been called "airway disease type", all of which is understood to be encompassed by the target of the present invention.

Most of the airway disease type is recognized to be cases where the surface of the airway becomes inflamed, and secretes mucus in a large volume. Further, the wall of the airway becomes thickened, the airway is narrowed, and thus the air passage is damaged.

Pulmonary emphysema refers to those diseases in which chronic inflammation causes disruption and fusion of alveoli pulmonis, and thus the lung contains air resulting in blistered or ballooned lung tissue pushing on the airway, and thus the airway is disrupted and the air passage becomes damaged.

Regardless of the type of the above mentioned two diseases, cells in the lung have been disrupted, and thus no radical treatment except for regeneration of cells in the lung can be assumed. The present invention should be noted for providing for the first time in history a radical treatment for COPD.

The diagnosis of COPD is performed as follows:

The diagnosis of COPD can be conducted with a simple respiratory function test (spiro test) using an apparatus called "spirometer". The spiro test is a test in which lung capacity and ease of airway passage in expiration are conducted.

In the spiro test, a "forced vital capacity (FVC)", which refers to the volume of respiration when a subject inspires as much as possible followed by intensive expiration, and a "volume within a second (FEV1.0)", which is a volume of expiration within the first second after the inspiration, are measured. Then, if the valued in which FEV1.0 value is divided by FVC value, (hereinafter one second rate "FEV1.0%") is less than 70%, then it is deemed that there is possibility of COPD. Inquiries are made to check conditions, symptoms, smoking history or state, living environment, and disease history and the like, and other tests will also be made, if necessary, such as, for example, imaging diagnosis, including chest X-ray examination, CT scan or the like, blood gas determination to study if the body lacks oxygen, and the like. This measures oxygen using blood from the artery or using pulse oxymeter, which is a small device for determination, equipped on the top of a finger and the like, to conduct the diagnosis of COPD.

If

$$\text{One second rate (FEV1.0\%) = One second volume/forced vital capacity X 100 < 70 \%,}$$
$$\text{then the subject is diagnosed with COPD.}$$

(Severity of COPD)

Treatment of COPD is performed, preferably depending on the extent of severity of disease. Therefore, the severity of COPD is determined as necessary. The severity of COPD is classified based on the % expectation one second volume (%FEV1.0) measurable by spiro test.

## TABLE 1

| severity | Characterization |
|---|---|
| state with risk | Spirometry: normal<br>chronic symptoms (cough, sputum) |
| Mild | FEV1.0 / FVC<70%<br>FEV1.0≧80% expected value<br>with/without chronic symptoms (cough, sputum) |
| Intermediate | FEV1.0 / FVC<70%<br>50%≦FEV1.0<80% expected value<br>with/without chronic symptoms (cough, sputum) |
| Severe | FEV1.0 / FVC<70%<br>30%≦FEV1.0<50% expected value<br>with/without chronic symptoms (cough, sputum) |
| Most severe | FEV1.0 / FVC<70%<br>FEV1.0<30% expected value or<br>FEV1.0<50% expected value with chronic respiratory failure |

FEV1.0: one second volume; FVC: forced vital capacity
GOLDCOPD global strategy for diagnosis, control and prevention (2003, revision)

As used herein the term "asthma" refers to a condition in which the respiratory system (for example, the airway (nasal cavity, pharynx, larynx, trachea, bronchus), and the lung) has enhanced responsiveness to mucous-related emphysema, viscous secretions and any other stimulus, resulting in the narrowing of the airway causing expiratory dyspnea. Asthma includes, but is not limited to, for example, bronchial asthma, infantile asthma, allergic asthma, atopic asthma, steroid refractory asthma, non-allergic asthma, endogenous asthma, exogenous asthma, aspirin asthma, cardiac asthma and infectious asthma and the like.

As used herein the term "rhinitis" refers to any inflammatory disorder of the nasal cavity mucous membrane. Rhinitis includes, but is not limited to, for example, allergic rhinitis, pollinosis, acute rhinitis, chronic rhinitis, hypertrophic rhinitis, chronic sinusitis (empyem), deflected septum and the like. Allergic rhinitis is a rhinitis which is associated with frequent sneezing, large volume of nasal hydrorrhea and blockage by edematous hyperplasia of the nasal mucous, as its main symptoms, and the allergens therefor (antigen) include house dust, dust mites, pollen of cedars or others, and the like, and it is said that in Japan there are fifteen million patients.

In practicing the pharmaceutical composition and method of the present invention, in the case where the patient is a human, preferably, prior to tests with a human, *in vitro,* subsequently in vivo, and finally animal tests are conducted to determine appropriate dosage amounts for desired therapy or prevention. The effects of a composition against a cell line and/or an animal model as precautionary test, may be determined using known technologies in the art. Accordingly to the present invention, in vitro assays which may be used for determining whether or not a specific compound can be administered, include observing binding between a transcriptional factor and a transcriptional binding sequence, and the like. In animal level tests, determination may be made by administering the composition at issue to such an animal as in the human, and confirming the level of therapeutic effects, e.g., observing changes in eosinophils. It should be understood in the art that once therapeutic and/or preventive effects are determined in an animal model, similar effects will usually be attained in a human.

The pharmaceutical composition of the present invention may be used in such a form that the decoy is taken into cells in an affected part or cells in an intended tissue.

The pharmaceutical composition of the present invention may be administered in any aseptic biocompatible pharmaceutical carrier (including, but not limited to, physiological saline, buffered physiological saline, dextrose, and water). The above-described decoy compound is used in a pharmaceutical composition mixed with an appropriate excipient, an adjuvant, and/or a pharmaceutically acceptable carrier. Such a composition may be administered to patients alone or in combination with another pharmaceutical agent in a pharmaceutical composition. In an embodiment of the present invention, the pharmaceutically acceptable carrier is pharmaceutically inactive.

The administration of the pharmaceutical composition of the present invention is achieved orally or parenterally. Parenteral delivery methods include topical, intra-arterial (e.g., directly into a tumor, aneurysm, etc.), intramuscular, subcutaneous, intramedullary, into the subarachnoid space, intraventricular, intravenous, intraperitoneal, or intranasal administrations. In addition to a decoy compound, the pharmaceutical composition of the present invention comprises a pharmaceutically acceptable carrier, such as an excipient and other compounds for accelerating the processing of the decoy compound so as to prepare a pharmaceutically acceptable formulation. Further details of techniques for prescription and administration are described in, for example, the latest version of "REMINGTON'S PHARMACEUTICAL SCIENCES" (Maack Publishing Co., Easton, PA).

A pharmaceutical composition for administration may be prepared using a pharmaceutically acceptable carrier well known in the art in a form suitable for administration. Such a carrier can be prepared as a tablet, a pill, a sugar-coated agent, a capsule, a liquid, a gel, a syrup, a slurry, a suspension, or the like, which is suitable for the patient to take the pharmaceutical composition. Pharmaceutically acceptable carriers include, but are not limited to, liposomes, lactose, trehalose, sucrose, mannitol, xylitol, crystalline cellulose, chitosan, calcium carbonate, talc, titanium oxide, or silica (silicon oxide) or the like.

The pharmaceutical composition for use may be obtained, for example, in the following manner: an active compound is combined with a solid excipient, the resultant mixture is pulverized if necessary, an appropriate compound is further added if necessary to obtain a tablet or the core of a sugar-coated agent, and the granular mixture is processed. The appropriate excipient may be a carbohydrate or protein filler, including, but not being limited to, the following: sugars including lactose, sucrose, mannitol, or sorbitol; starch derived from maize (corn starch), wheat, rice, potato, or other plants; cellulose such as methylcellulose, hydroxypropylmethylcellulose, or sodium carboxymethylcellulose; and gum including gum Arabic and gum tragacanth; and protein such as gelatin and collagen. A disintegrant or a solubilizing agent such as crosslinked polyvinyl pyrrolidone, agar, alginic acid or a salt thereof (e.g., sodium alginate) may be used if necessary.

The sugar-coated agent core is provided along with an appropriate coating, such as a condensed sugar solution. The sugar-coated agent core may also contain gum arabic, talc, polyvinyl pyrrolidone, carbopolygel, polyethylene glycol, and/or titanium dioxide, a lacquer solution, and an appropriate organic solvent or a solvent mixed solution. To identify a product, or characterize the amount of an active compound (i.e., dose), dye or pigment may be added to tablets or sugar-coated agents.

The pharmaceutical preparation which may be used may contain, for example, a soft sealed capsule consisting of a gelatin capsule, gelatin and coating (e.g., glycerol or sorbitol). The gelatin capsule may contain an active component mixed with a filler or binder such as lactose or starch, a lubricant such as talc or magnesium stearate, and optionally a stabilizer. In the soft capsule, the decoy compound may be dissolved or suspended in an appropriate liquid, such as a fatty oil, liquid paraffin or liquid polyethylene glycol, with or without a stabilizer.

The pharmaceutical preparation for parenteral administration contains an aqueous solution of an active compound. For the purpose of injection, the pharmaceutical composition of the present invention is prepared in an aqueous solution, preferably Hank's solution, Ringer's solution, or a physiologically suitable buffer such as a buffered physiological saline. The aqueous suspension for injection may contain a substance for increasing the viscosity of a suspension (e.g., sodium carboxymethylcellulose, sorbitol, or dextran). Further, the suspension of the active compound may be prepared as an appropriate oily suspension. Appropriate lipophilic solvents or vehicles include fatty acids such as sesame oil, synthetic fatty acid esters such as ethyl oleate or triglycerides, or liposomes. The suspension may contain a stabilizer which allows a high-concentration solution preparation, or an appropriate pharmaceutical agent or a reagent for increasing the solubility of the compound, if necessary.

For topical administration, such as lung administration, transairway administration or intranasal administration, an appropriate penetrant for the specific barrier to be penetrated may be used in the preparation. Such a penetrant is generally known in the art.

The pharmaceutical composition of the present invention may be produced using a method similar to methods known in the art (e.g., conventional mixing, dissolution, rendering to granules, preparation of a sugar-coated agent, elutriation, emulsification, capsulation, inclusion, or freeze drying).

Accordingly, it is understood that a method for adapting to the administration to the respiratory system may be readily carried out in view of the known technologies in the art. For example, it should be understood that it is preferable that intra-airway administration or transairway absorption may be adapted to a form of spray or expiration adapted format including microparticles such as dry powder. As used herein, for example, the present invention is adapted to administration by metered dose inhaler (MDI), dry powder inhaler (DPI) or nebulizer. For nasal cavity administration, it should be understood that aqueous solution is also suitable. For the pulmonary administration, it should be understood that it is preferable that the similar dosage to the airway administration or even more fine microparticles are used. Dry powder may be manufactured by means of one selected from the group consisting of a bowl mill, a bead mill, a jet mill, an ultimizer, a mortar, a stonemill, spray drying and supercritical fluid. For airway administration, it is desirable that the aerodynamic average particle size of dry powder is typically about 0.01 to about 50 $\mu$m, preferably, about 0.1 to about 30 $\mu$m, still preferably about 0.1 to about 10 $\mu$m. For pulmonary administration, in view of the delivery into the alveoli pulmonis, those particles having an aerodynamic average particle size of about 3 $\mu$m or less are preferably manufactured, but the present invention is not limited thereto. The term "aerodynamic average particle size" refers to a particle size for which the sedimentation rate is the same as the particle in issue and the density thereof is 1 (g/cm$^3$) and has a spherical form. Such a size may be determined based on the difference between the sedimentation distance of the particles, or the difference between inertia when accelerating the particles. For example, cascade impactors and the like are to capture particles by grading such particles. The reasons why the present invention employs such aerodynamic average particle sizes is based on the theory where the ratio of the particles deposited to each site by aspirated into the body by respiration may be determined by its aerodynamic average particle size. Typically, dynamic light scattering (for example, using LB-550 available from HORIBA), or laser diffraction (for example, using LB-500 available from HORIBA), centrifugation sedimentation (for example, CPA-500 available from HORIBA) and the like may be used for determination. As used herein, the term "dynamic light scattering" refers to a method for measuring a particle size by radiating laser light to a particle in a solution, and obtaining a Brownian motion rate (dispersion efficiency) from temporal fluctuation of output scattered light. Laser diffraction method is a method for obtaining particle size by means of the diffraction phenomenon of light (Fraunhofer phenomenon) and Mie scattering phenomenon. Centrifugation sedimentation permeation method is a method for measuring a particle size from a relationship between the size of the particle which sediments in a medium and the sedimentation speed.

Preferably, in the case of parenteral administration, such as topical administration to the cell of an affected part or the cells of an intended tissue, the pharmaceutical composition of the present invention may contain a synthetic or naturally-occurring hydrophilic polymer as a carrier. Examples of such a hydrophilic polymer include hydroxypropylcellulose and polyethylene glycol. The decoy compound of the present invention may be mixed with the above-described hydrophilic polymer in an appropriate solvent. The solvent may be removed by a method such as air drying. The resultant compound may be shaped into a desired form, such as sheet, and then may be given to a target site. Such a preparation containing a hydrophilic polymer has a low moisture content, an excellent shelf life, and has excellent retention of the decoy compound since the preparation absorbs water and turns into gel when used.

Such a sheet may include a hydrophilic sheet obtained by mixing polyhydric alcohol with a compound similar to the above-described composition components, such as cellulose or starch, or a derivative thereof, a synthetic polymer compound or the like and adjusting the hardness of the sheet.

Such a sheet may be placed in a target site under a laparoscope. Currently, laparoscopic surgery has been dramatically developed as a non-invasive technique. By combining the pharmaceutical composition of the present invention with the laparoscope technique, a method for treatment of diseases, which can be repeatedly used, may be provided.

Alternatively, when a nucleic acid or a modification thereof is employed as a decoy, the pharmaceutical composition of the present invention is advantageously used in a form which is generally used in gene introduction methods, such as a membrane fusion liposome preparation using Sendai virus (HVJ) or the like, a liposome preparation using endocytosis or the like, a preparation containing a cationic lipid such as Lipofectamine (Lifetech Oriental) or the like, or a viral preparation using a retrovirus vector, an adenovirus vector, or the like. Particularly, a membrane fusion liposome preparation is preferable.

The liposome preparation is any of the following liposome construct: a large unilamellar vesicle (LUV), a multilammelar vesicle (MLV), and a small unilamellar vesicle (SUV). The LUV has a particle system ranging from 200 to 1000 nm. The MLV has a particle system ranging from 400 to 3500 nm. The SUV has a particle system ranging from 20 to 50 nm. The membrane fusion liposome preparation using Sendai virus or the like preferably employs MLV having a particle system ranging from 200 nm to 1000 nm.

There is no particular limitation on a method for producing liposomes as long as the liposomes can hold a decoy. The liposomes can be produced by a commonly used method, such as, for example, a reversed phase evaporation method (Szoka, F et al., Biochim. Biophys. Acta, Vol. 601 559(1980)), an ether infusion method (Deamer, D.W.: Ann. N.Y. Acad. Sci., Vol. 308 250(1978)), a surfactant method (Brunner, J et al.: Biochim. Biophys. Acta, Vol. 455 322(1976)), or the like.

Examples of lipids for forming liposomes include phospholipids, cholesterols, nitrogen lipids, and the like. Generally, phospholipids are preferable, including naturally-occurring phospholipids, such as phosphatidylcholine, phosphatidylserine, phosphatidylglycerol, phosphatidylinositol, phosphatidylethanolamine, phosphatidic acid, cardiolipin, sphingomyelin, egg yolk lecithin, soybean lecithin, lysolecithin, and the like, or the corresponding phospholipids hydrogenated by a commonly used method, and in addition, synthetic phospholipids, such as dicetylphosphate, distearoylphosphatidylcholine, dipalmitoylphosphatidylcholine, dipalmitoylphosphatidylethanolamine, dipalmitoylphosphatidylserine, eleostearoylphosphatidylcholine, eleostearoylphosphatidylethanolamine, eleostearoylphosphatidylserine, and the like.

The lipids, including these phospholipids, can be used alone or with at least two in a combination. In this case, lipids containing within the molecule an atomic group having a cationic group, such as ethanolamine, choline, or the like, can be used to increase the binding rate of a negatively charged decoy nucleic acid. In addition to the major phospholipids used to form liposomes, an additive, such as cholesterols, stearylamine, α-tocopherol, or the like, which are generally known as an additive for the formation of liposomes, can be used.

The thus-obtained liposomes can additionally contain a substance for promoting membrane fusion, such as a membrane fusion promoting protein purified from Sendai virus, inactivated Sendai virus, or the like, so as to accelerate uptake into cells at an affected site or cells in an intended tissue.

An exemplary method for producing a liposome preparation will be specifically described below. For example, the above-described substance for forming a liposome is dissolved along with cholesterol in an organic solvent, such as tetrahydrofuran, chloroform, ethanol, or the like. The resultant solution is put into an appropriate vessel, followed by removal of the solvent under reduced pressure, thereby forming a film of the liposome forming substance on an inside wall of the vessel. A buffer solution containing a decoy is added to the vessel followed by agitation. The above-described membrane fusion promoting substance is added to the resultant liposome if necessary, followed by isolation of the liposome. The thus-obtained liposome containing the decoy can be suspended in an appropriate solvent or can be freeze-dried and thereafter dispersed in an appropriate solvent. The resultant suspension can be used in treatment. The membrane fusion promoting substance may be added in the interim period after isolation of the liposome and before use.

As used herein the term "treatment" or "therapy" refers to the prevention of deterioration of a disease or disorder when a patient contracts such a disease or disorder, preferably, at least maintenance of the status quo, and more preferably, alleviation, still more preferably, resolution.

As used herein the term "prophylaxis" or "prevention" refers to, when referring to a disease or disorder, a type of treatment conducted before such a condition occurs such that such a condition will not occur. Accordingly, it includes, with respect to a disease or disorder, prevention, delay or the like of such a condition, and prevention of deterioration.

As used herein, the term "treatment" in the broadest sense, with respect to a disease, disorder or condition, refers to any medical act thereto, and include any act for diagnosis, therapy, prevention, prognosis and the like.

Generally, drugs used for drug therapy for treating a disease of the respiratory system such as asthma, are divided into relievers, which are attack treating drugs for alleviation of an attack, and controllers, long-term controlling drugs for attack prevention. It is understood that NF-κB can be used for both prevention and therapy. This is because, in particular, attack prevention is substantially the therapy of the asthma *per se,* and it is more important for having substantial therapeutic effects, rather than an attack reliever. Once it is understood that the present invention can be used for other diseases such as rhinitis, COPD and the like, it can also be used for therapy. On the other hand, once it is understood that the present invention can be used for therapy, then it can also be used for prevention. Accordingly, it is understood that in the present invention, when an appropriate dosage is known for prevention or therapy, those skilled in the art will be able to calculate an appropriate dosage for either therapy or prevention from the known dosage.

The pharmaceutical composition of the present invention includes a composition containing an effective amount of decoy compound which can achieve the intended purpose of the decoy compound. "Preventively effective amount", "therapeutically effective amount" and "pharmacologically effective amount" are terms which are well recognized by those skilled in the art and which refer to an amount of a pharmaceutical agent effective for the production of an intended pharmacological effect such as a preventive or therapeutic effect. Therefore, the preventively effective amount is an amount sufficient to avoid developing the symptom, and the therapeutically effective amount is an amount sufficient to reduce the manifestation of the disease to be treated. A useful assay for confirming an effective amount (e.g., a therapeutically effective amount) for a predetermined application is to measure the degree of recovery from a target disease. An amount actually administered depends on an individual to be treated. The amount is preferably optimized so as to obtain a desired effect without significant side effects. The determination of a therapeutically effective dose is within the ability of those skilled in the art.

A preventively or therapeutically effective dose of any compound can be initially estimated using either a cell culture assay or any appropriate animal model. The animal model is used to achieve a desired concentration range and an administration route. Thereafter, such information can be used to determine a dose and route useful for administration into humans.

Preventively and therapeutically effective amounts refer to an amount sufficient prevent the incidence of a disease, and an amount of a decoy compound which results in the amelioration of symptoms or conditions of a disease, respectively. The therapeutic effect and toxicity of such a compound may be determined by standard pharmaceutical procedures in cell culture or experimental animals (e.g., $ED_{50}$, a dose therapeutically effective for 50% of a population; and $LD_{50}$, a dose lethal to 50% of a population). The dose ratio between therapeutic and toxic effects is a therapeutic index, and it can be expressed as the ratio of $ED_{50}/LD_{50}$. Pharmaceutical compositions which exhibit high therapeutic indices are preferable. The data obtained from cell culture assays and animal studies can be used for formulating a dosage range for use in humans. The dosage of such compounds lies preferably within a range of circulating concentrations that include the $ED_{50}$, with little or no toxicity. Such a dosage may vary within this range depending upon the dosage form employed, the susceptibility of a patient, and the route of administration. As an example, the dose of a decoy is appropriately selected depending on the age and other conditions of a patient, the type of a disease, the type of the decoy employed, and the like. For example, in the case of intravascular administration, intramuscular administration, intra-articular administration, or application to the skin, 1 $\mu$g to 100 mg can be generally administered once a day to several times a day.

The exact dose is chosen by an individual physician in view of the condition of a patient to be treated. Doses and administration are adjusted to provide a sufficient level of the active portion, or to attain a desired effect. Additional factors to be considered include the severity of the condition of a disease (e.g., the size and location of a tumor); the age, weight and sex of a patient; a dietary restriction time and frequency of administration, potential drug interactions, reaction susceptibility, and resistance/response to treatment). A pharmaceutical composition with sustained action may be administered every 3 to 4 days, every week, or once every two weeks, depending on the half life and clearance rate of a specific preparation. Guidance for specific doses and delivery methods are provided in publications known in the art.

A pharmaceutical product containing a decoy thus produced as a major ingredient, may be administered by means of a variety of methods depending on the type of diseases, type of decoy to be used, and the like. For example, in the case of asthma, COPD and the like, atomizer type inhalators such as MDI, BDI, or nebulizers and the like may be used for inhalation. For example, in the case of rhinitis, absorption and inhalation may be used for administration.

A method for inhalation conventionally used as a method for administration via the nasal cavity, airway and nasal pathways and the like will be described below. In intra-airway administration formulations, transairway absorption formulations or pernasal absorption formulations, it is usually preferable to make a drug solution in a mist form or as fine powder (dry powder). Generally, a drug solution formed may be inhaled by means of a nebulizer, those processed into powder may be inhaled by means of a gas-atomizing type, MDI (metered dose inhaler) or expiration inhalation system, DPI (dry powder inhaler) with the drug loaded thereinto.

With respect to powder inhalers, there are two types presently used for rapid and deep inhalation, "dry powder inhaler (DPI)" and delayed inhaling type "metered dose inhaler (MDI)".

DPI are further classified into three categories: Multi-dose reservoir (product name; Turbuhaler, available from AstraZeneca), Multi-unit dose (product name; Accuhaler/Diskus, available from GSK), Unit dose (many manufacturers).

Inhaler refers to a kit consisting of a mouth piece and cartridge (tube), and are usually employed by sealing both termini of the tube with aluminum foil. Prior to use, tube is equipped with the mouth piece to pierce the aluminum foil, thereby allowing inspiration of powdered drug inside.

On the other hand, absorption of a drug solution may be achieved by means of a nebulizer or respirator, an artificial respirator. A drug aerosol caused by the nebulizer is slow in the speed and flows in the air, and thus it is easy for any one to absorb the drug, which is advantageous.

A bead mill is an apparatus for grinding and dispersal, provided by loading beads into a vessel, rotating the central rotating axis to move the beads, delivering material to the beads and grinding the beads.

A jet mill is an apparatus for grinding particles by means of a high pressure airflow, by accelerating the particles to a speed of about sound velocity in the air flow to cause collisions that grind the particles.

A ultimizer is a device for microparticulization by colliding powder bodies into each other at an extreme speed using pressurized atomization or pressurized slurry material, by means of a gas such as air, nitrogen, or the like.

Microparticulization by ultracritical fluid is a method for producing microparticles by means of spray drying a drug, solubilized in a solvent, by rapid expansion from a nozzle into a pressurized vessel, and solubilized into ultracritical $CO_2$.

The average particle size of dry powder used in the present invention is not limited, but is usually from about 0.01 to about 50 $\mu$m, and preferably, about 0.05 $\mu$m to about 30 $\mu$m and still more preferably from about 0.1 $\mu$m to about 10 $\mu$m.

When administering in a site selective manner, in order to act on the trachea, it generally requires an aerodynamic average particle size of about 10 to about 30 micrometer, in order to act on the bronchus, it generally requires an aerodynamic average particle size of about 3 to about 10 micrometer, in order to act on the alveoli pulmonis, it generally requires an aerodynamic average particle size of about 3 $\mu$m or less, preferably an aerodynamic average particle size of about 2 $\mu$m or less, and preferably requires a homogenous particle size.

Dosages of NF-$\kappa$B of the present invention is not limited to a particular value, but when administered as a dry powder for intraairway administration or transairway inhalation, the dosage is usually, per adult, 10$\mu$ to 100mg per round, and preferably 50$\mu$g-50mg per round still more preferably, 10mg or less per round. When administered as transnasal inhalation, in particular as a nasal drop for rhinitis, the dosage is usually, per adult, 10$\mu$ to 100mg per round, and preferably 50$\mu$g-50mg per round still more preferably, 10mg or less per round. The dosage appropriately varies depending on the targeted disease, condition (extent), age, and the presence or absence of complication(s).

Further, the NF-$\kappa$B decoy of the present invention may be administered in a naked form, or may be administered in a variety of vector, such as an HVJ-envelope vector, with the decoy enclosed therein.

In the manufacture of dry powders of the present invention, an excipient is usually used.

Excipients which can be used are those that are inactive against NF-$\kappa$B and as long as the use is recognized as a pharmaceutical additive, no limitation is made for such excipient, and include preferably for example, monosaccharides such as galactose, mannose, sorbose; disaccharides such as lactose, sucrose and trehalose and the like; polysaccharides such as starch, raffinose, dextran and the like; sugar alcohols (including glycerol, erythritol, arabitol, xylitol, sorbitol, mannitol), glycols (including ethylene glycol, propylene glycol, polyethylene glycol, polypropylene glycol), cellulose-like polymers (including hydroxy cellulose, hydroxy propyl cellulose), insoluble additives (crystalline cellulose, chitosan, calcium carbonate, talc, titanium oxide or silica (silicon oxide) or a mixture thereof.

Aerosol systems in the form of spray includes, for example, vessels with propellant included therein, aerosol systems without propellant which are also under development. Further, in an aerosol system the propellant comprises an active component together with conventional additives such as lactose. A composition consisting of these components, determines the properties of the aerosol inhalant, that is, for example, particle distribution, delivery rate, viscosity and the like.

Aerosols may be obtained as a two-phase aerosol (i.e. air and liquid) or three-phase aerosol (air, liquid and solid or liquid). Two-phase aerosols consists of a solution of active substance dissolved in liquefaction propellant and spray propellant. Solvents include for example, propellant or a mixture of propellant and alcohol or propylene glycol or the like. Polyethylene glycol is used for improving the solubility of active substances.

Three-phase systems include, a suspension or emulsion each including one or more active ingredients together with a mist propellant. The suspension includes an active substance dispersed in a propellant system by means of conventional additive such as a wetting agent and/or solid carrier such as talc or colloidal silica. If possible, a known propellant such as a carbohydrate is used so as to prevent damage to the ozone layer.

Inhalation is only possible if the solution for inhalation is atomized to very small particles of a size range of 1-10 $\mu$m. It is preferable to inhale a aerosol finely distributed therein. In the case of conventional aerosols other than the above, it is preferable not to inhale. In order to obtain detailed information relating to aerosol inhalants, refer to, for example, a pharmacopoeia such as the Japanese Pharmacopoeia, US Pharmacopoeia and the like, which is herein incorporated hereby as reference in entirety.

Liposomes used according to the present invention preferably are a size of the range of 0.1$\mu$m-2$\mu$m, particularly preferably 0.4 $\mu$m. In particular, the use of unilamellar liposome is preferable. Preparation of liposomes may be achieved by means of an extrusion fabrication apparatus or high pressure filtration. For example, liposome may be prepared by reconstituting a dry lipid film in a desired liquid (for example, Parthasarathy et al., Cancer Chemother. and Pharmacol. 1999, 43(4), 277-283). According to this, so-called multi-lamellar liposome (i.e. a liposome having a multiple of lipid layers surrounding aqueous core) is produced. Such a liposome is extremely homogenous in size.

In another aspect, the present invention provides a system for the prevention or treatment of a lung tissue, comprising means for pulmonary administration and a NF-$\kappa$B decoy in a format adapted for lung administration. It should be understood that such a system may be in any format as long as the system is for facilitating the pulmonary administration of the agent. Such a means for achieving pulmonary administration includes, but is not limited to, for example, an inhalation device, bronchoscope and the like.

In a preferable embodiment, the NF-$\kappa$B decoy used may be in any form such as a dry powder, liposomes, microparticles and the like.

In one embodiment, the NF-$\kappa$B used in the composition of the present invention present is at an effective concentration. Such a concentration may be determined in a simple in vitro experiment. Such an experiment includes but is not limited to for example, a method for determining NF-$\kappa$B decoy labeled with radioisotope (for example, I$^{125}$) by means of scintigram and the like. Preferably, it is advantageous to use scintigram adapted for clinical use. This is because it can be used for confirmation using large animals such as a dog.

In a preferable embodiment, NF-$\kappa$B decoy used may be administered to a desired tissue such as a lung tissue. Such a selective administration may be achieved by a preferable means described as means for preferable administration in the system of the present invention such as inhaler or bronchoscope. Accordingly, more preferably, the NF-$\kappa$B decoy

is not substantially administered to tissues other than the desired tissue such as the lung. If NF-κB decoy is administered to the tissues other than the desired tissue, it may be possible to cause an unexpected event resulting in the expression of side effects.

The lung administration system which may be used, is any system which is capable of delivering the NF-κB decoy of the present invention to the lung. Such a lung administration system includes inhaler or bronchoscope and includes those bronchoscope systems commercially available from Olympus Inc.

Hereinafter, the present invention will be described by way of examples. These examples are for illustrative purposes only. The present invention is not limited by these examples or the detailed description of the invention hereinabove, except as by the appended claims.

EXAMPLES

Hereinafter the present invention is described by ways of Examples, which only exemplify the present invention but are not intended to be limiting.

(EXAMPLE 1: Preparation of a decoy)

The following decoy oligodeoxynucleotides have been prepared.

The sequence of the decoy used are as follows:

Decoys used herein:

```
NF-κB decoy (SEQ ID NO. 1)
5'-CCT-TGA-AGG-GAT-TTC-CCT-CC-3'
3'-GGA-GGG-AAA-TCC-CTT-CAA-GG-5'


NF-κB scrambled decoy (SEQ ID NO. 2)
5'-TTG-CCG-TAC-CTG-ACT-TAG-CC-3'
3'-GGC-TAA-GTC-AGG-TAC-GGC-AA-5'
```

```
NF-κB decoy variants (SEQ ID NO: 3)
5'-GGG(A/G)(C/A/T)T(T/C)(T/C)(C/A/T)C—3' (SEQ ID NO:
3)
```

```
3'-CCC(T/C)(G/T/A)A(A/G)(A/G)(G/T/A)G—5'
```

These decoy solutions were stored at -20 °C until use at 100mg/vial.

(Example 2: NF-κB decoy treatment using a rat model of asthma)

Next, an asthma model was produced to demonstrate the effects of the decoy according to the present invention. An asthma model was produced, substantially based on a method described in Eur J Pharmacol. 1995 Dec 7;293(4): 401-12.

(Animals and methods for sensitization)

Brown Norway rats (8-10 weeks old; weight: 200-300 g) were obtained from Charles River, Japan. Rats were bred and maintained based on the provisions defined by Osaka University in the spirit of animal welfare. These rats were given 4 mg of aluminum hydroxylate and 1 mg ovalbumin (OVA; Sigma, grade V) in one ml of pyrogen-free saline by cervix subcutaneous injection for sensitization. Bordetella pertussis vaccine containing $3 \times 10^9$ heat inactivated bacteria was used for intraperitoneal injection as an adjuvant. As control, the same solution as mentioned above except for the omission of ovalbumin was intraperitoneally injected, thus providing a negative control.

(Preparation of Sendai Virus Envelope Vector)

Sendai virus (HVJ) envelope vector (HVJ-E) was prepared as follows. Briefly, virus suspension ($1.0 \times 10^4$ hemagglutinating units (HAU)) was inactivated by UV irradiation (99 mJ/cm$^2$) and mixed with decoy oligonucleotide (200 μg) and 0.3% Triton-X. Following centrifugation, the inactivated virus suspension was washed with 1 ml of balanced salt solution (BSS; 10 mM Tris-Cl, pH 7.5, 137 mM NaCl, and 5.4 mM KCl) to remove surfactant and unincorporated oligonucleotides. Following centrifugation, the envelope vector was suspended in an appropriate amount of phosphate buffered saline (PBS). The vector was stored at 4°C until use.

(Experimental Protocol)

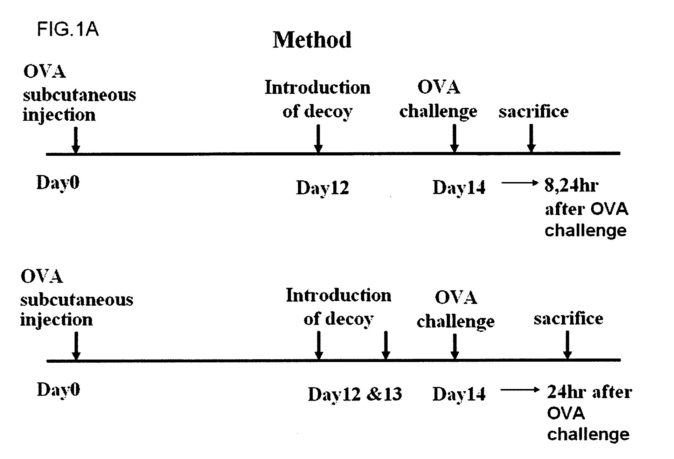

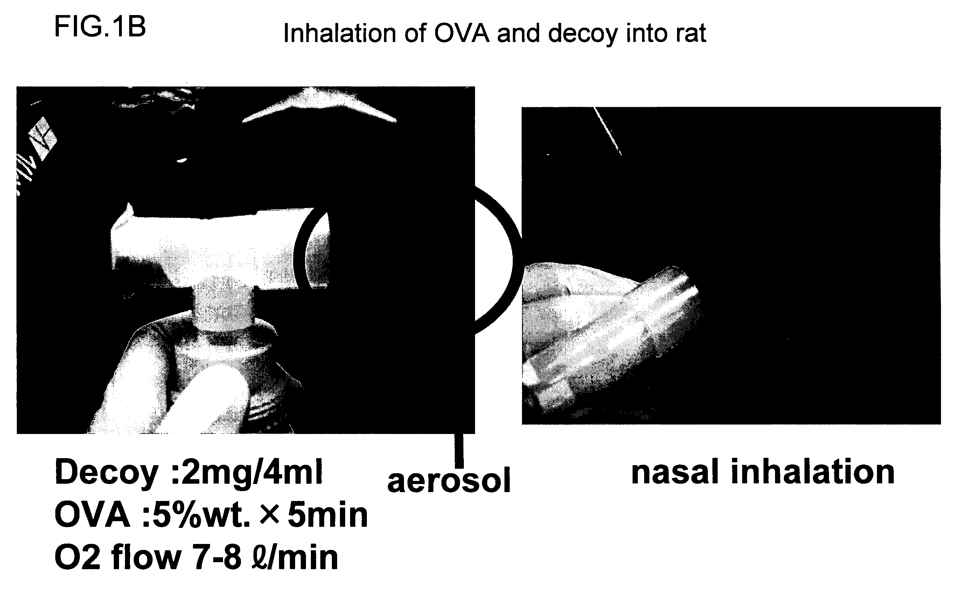

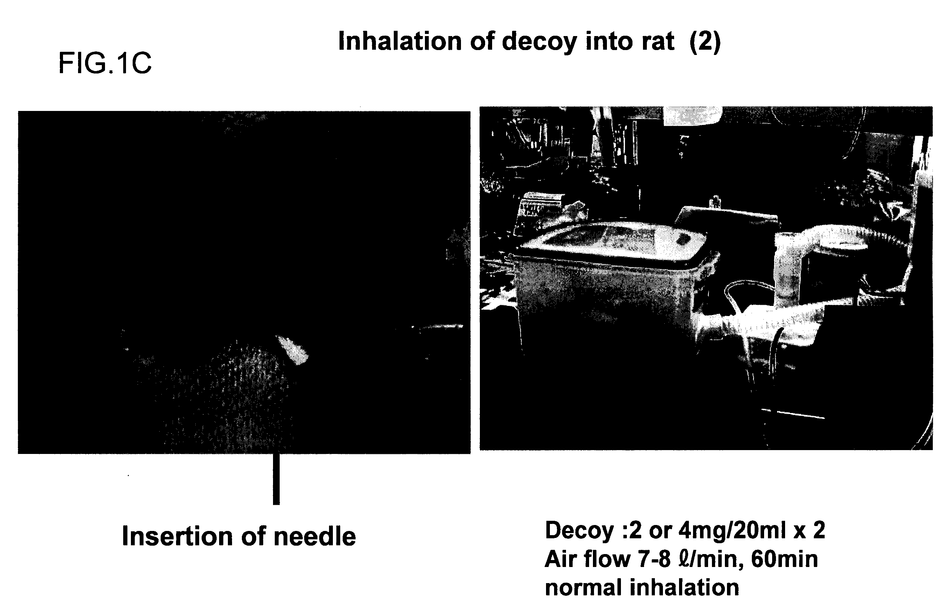

12 days after sensitization, the rats were treated as follows. Using orotracheal instillation, (1) 0.5 ml of physiological saline (control); (2) 500 μg of naked decoy/0.5 ml; (3) 200 μg of decoy treated with HVJ-E $1.0 \times 10^4$ HAU/0.5 ml; (4) 500 μg of decoy treated with HVJ-E $2.5 \times 10^4$ HAU/0.5 ml HVJ-E; (5) HVJ-E $2.5 \times 10^4$ HAU/0.5 ml, were administered, respectively. On day 14, the rats were challenged with 5% aerosolized ovalbumin for 5 min using a nebulizer (PARI turbo). The airflow rate was 7 to 8 liters/min. As a result, airway irritation was induced. A flowchart of the experimental protocol is shown in Figure **1.**

(Introduction of Decoy)

Decoys were administered into rats by nasal inhalation (using an aerosol) or intubation (liquid, aerosol, respirator).
In the first experiment, decoys were administered as follows: 2 mg $\times$ 1 time, 1 mg $\times$ 2 times, or 2 mg $\times$ 2 times (aerosol), or 0.5 mg $\times$ 1 time (liquid administration).
In the second experiment, decoys were prepared using HVJ-E as described above and were administered.

(Bronchial Alveolar Lavage (BAL))

BAL fluid was collected 24 hours after ovalbumin challenge. The rats were euthanized by an intraperitoneal injection of an overdose of sodium pentobarbital. BAL was performed using 5 ml of phosphate buffered saline (PBS; 137 mM NaCl, 10 mM sodium phosphate buffer pH 7.4, 2.7 mM KCl), 4 times. The total number of cells in the BAL fluid was determined using an erythrocytometer. Differential cell counts were performed by counting more than 300 cells with Diff-Quick stain (IBMC Inc., Chicago, IL: Catalog # K7124).

(Results)

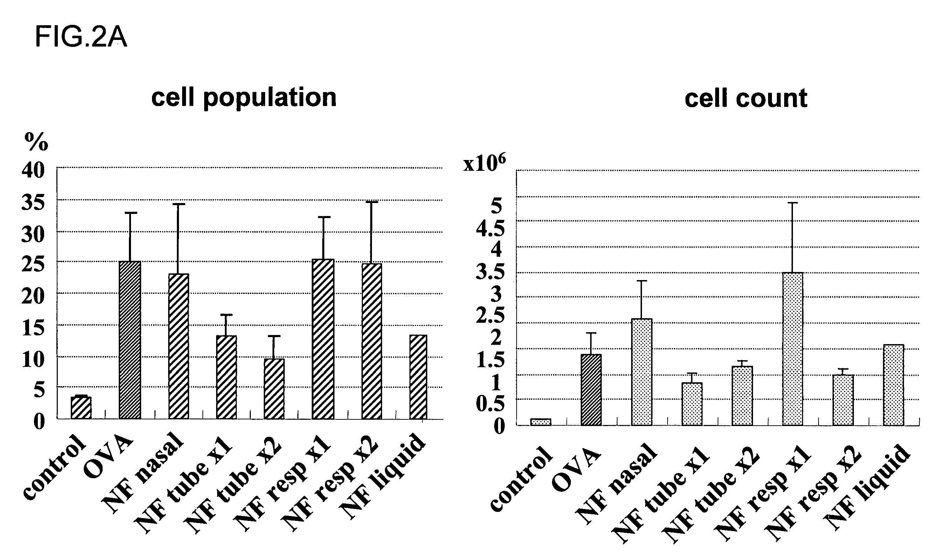

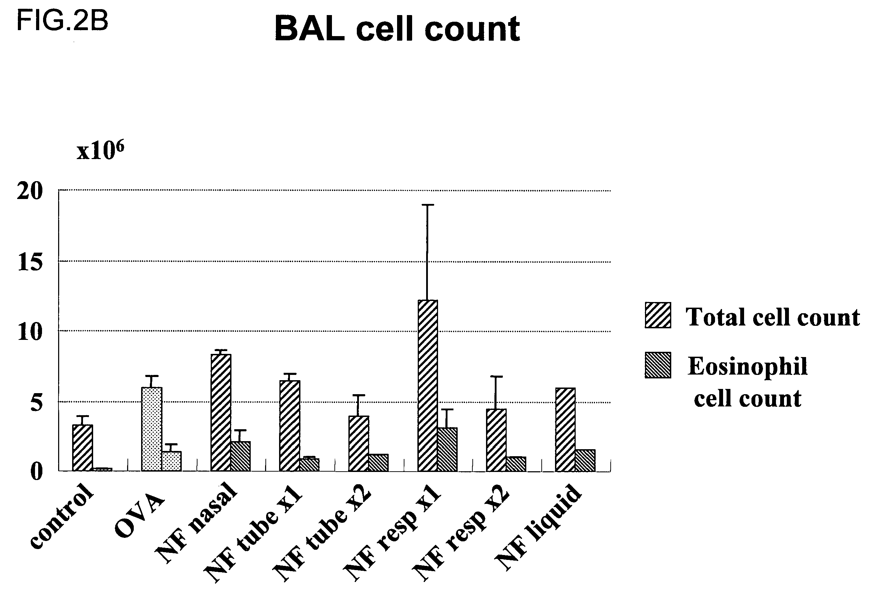

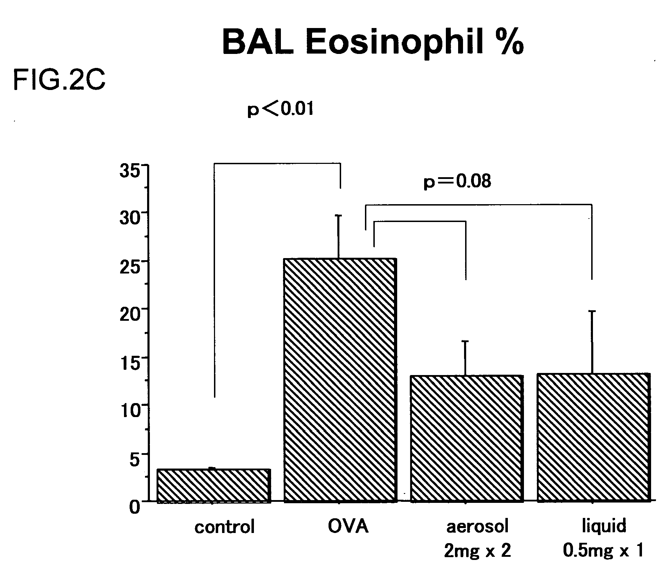

Figure **2** shows the behavior of the percentage of eosinophils in BAL in the first experiment. It was found that ovalbumin challenge elicited a statistically significant increase in the numbers of eosinophils ($p < 0.01$). After challenge, aerosol treatment or liquid treatment was performed. As a result, in either case, the increase of eosinophils was statistically significantly suppressed ($p = 0.08$ for each). Therefore, it was demonstrated that the decoy of the present invention is effective for diseases causing eosinophil abnormalities, such as asthma and the like, irrespective of administration method.

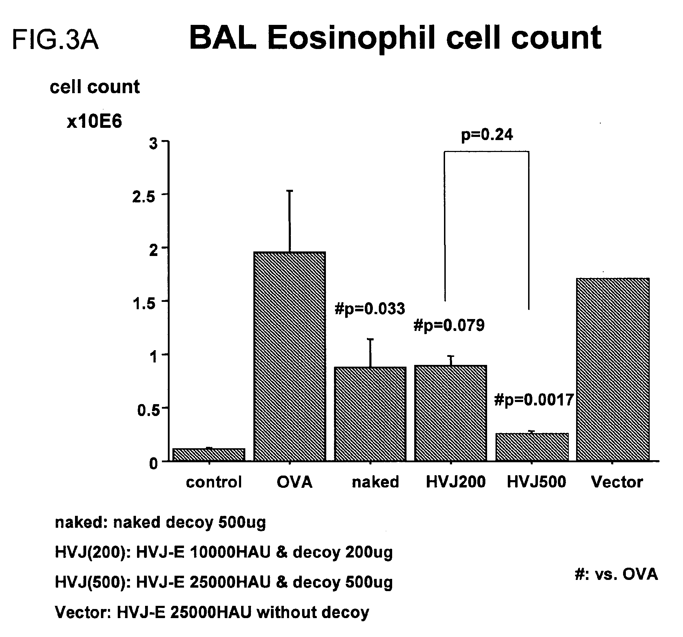

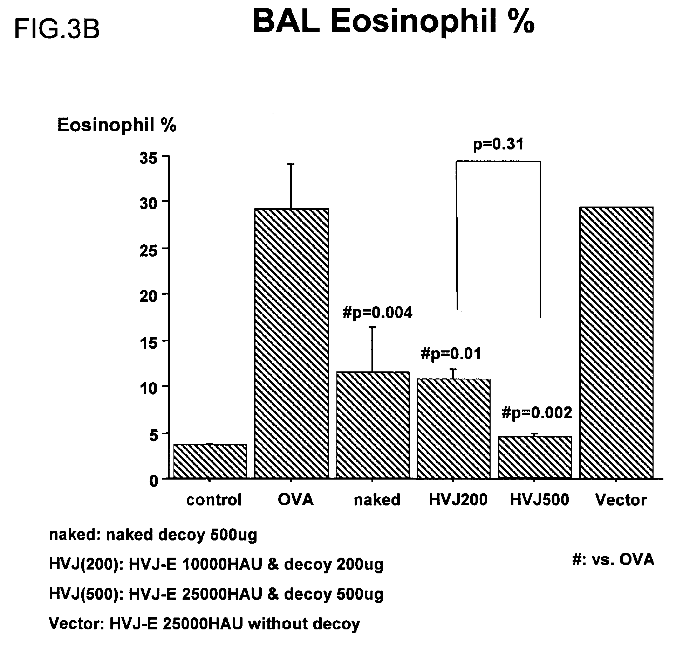

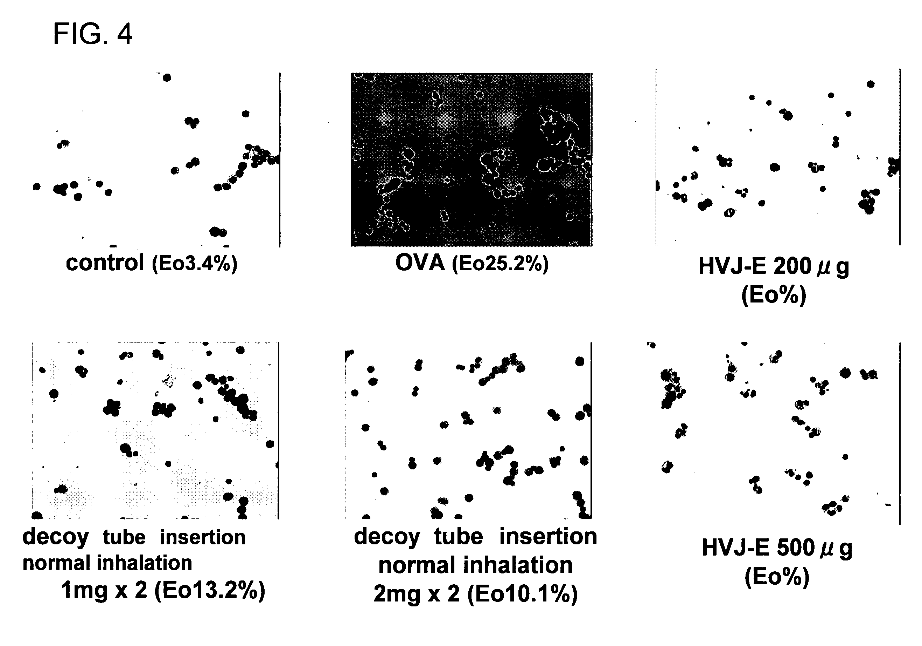

Figure **3** shows the effect of the decoy of the present invention when Sendai virus was used as another administration method. After ovalbumin challenge, treatment was performed using the above-described HVJ-E decoy prescription. As a result, the HVJ-E decoy had a significantly higher effect than the naked decoy (See Figure **4**). An HVJ-E formulation having a 2.5-fold amount had a statistically significantly higher therapeutic effect. It was demonstrated that such an HJV-E effect was not attributed to the vector itself. Thus, in the present invention, it was demonstrated that the HVJ-E formulation has a higher therapeutic effect.

(Example 3: Treatment with Variant)

Next, decoy variants were used in asthma model rats instead of the decoys used in the above-described examples. Whether or not the same effect can be obtained with decoy variants was confirmed. Here, as a variant, a consensus sequence of NF-κB, GGG (A/G) (C/A/T) T (T/C) (T/C) (C/A/T) C (SEQ ID NO. 3), was used. Therefore, it was demonstrated that any sequence recognized by NF-κB can be used to treat and prevent diseases, disorders and/or conditions caused by expression of a gene controlled by NF-κB, and diseases, disorders and/or conditions caused by an eosinophil abnormality.

The same experiments as in the above-described examples were conducted, except for the use of different decoys. For either aortic aneurysm or asthma, it was demonstrated that therapeutic and prophylactic effects were exhibited. Therefore, the present invention is not limited to the particular NF-KB decoys. It was demonstrated that any variant NF-KB decoy has the same therapeutic and prophylactic effects as long as the biological activity (i.e., activity to bind to a transcriptional factor sequence) is maintained.

(Example 4: Effects of NF-κB decoy against a rat asthma model)

Next, the effect of an NF-κB on the rat asthma model were elucidated. In the present example, a powder inhalation agent of a naked NF-κB decoy was administered.

(Preparation of NF-κB decoy powder inhalation agent)

NF-kB (100mg) was located in a transparent glass vessel (size 25.5 mm, height 48.5 mm) together with 18g of zirconia beads (diameter:3.0mm), and was ground in a bowl mill rotating cradle (Asahi Rika) at 200 rpm for 10 hours.

Lactose (50 g, product name: Pharmatose 450M available from DMV) was ground in a jet mill (product name: A-O jet mill, available from Seishin Enterprise).

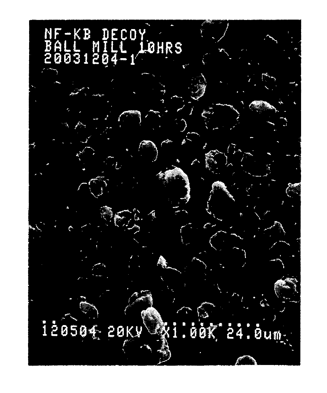

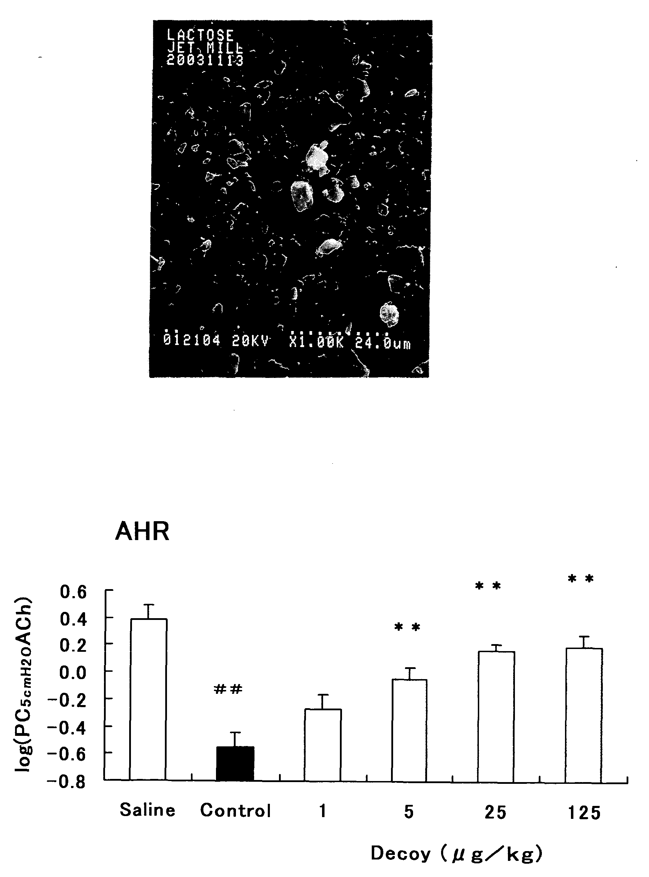

In order to determine the size of NF-κB decoy thus-obtained and lactose, scanning electron microscope (S-570, available from HITACHI) was used for observation. The results thereof are shown in Figure 5 (NF-kB decoy) and Figure 6 (lactose). The magnification of the photographs is x1000 and the size of each particle is less than 10 μm.

Generally, it is believed that it is desirable to use 10 μm or less for administration into the trachea, but the present invention is not limited thereto. Further, it is known that the use of those with 3μm or less allows administration into the alveoli of the lung.

Ground NF-κB decoy (100mg), ground lactose (3700mg) and light anhydrous silicic acid (Aerosil 200, available from Japan Aerosil Inc.; 200mg) were mixed in a small grinder (available from Shibata Kagaku Inc.) for one minute to yield an NF-κB decoy mixture powder (25μg/mg) .

NF-κB decoy mixture powder (25μg/mg; 1000mg), ground lactose (2850mg) and light anhydrous silicic acid (150mg) were mixed in the small grinder for one minute to yield an NF-κB decoy mixture powder (6.25μg/mg) .

NF-κB decoy mixture powder (6.25μg/mg; 800mg), ground lactose (3040mg) and light anhydrous silicic acid (160mg) were mixed in the small grinder for one minute to yield an NF-κB decoy mixture powder (1.25μg/mg).

NF-κB decoy mixture powder (25μg/mg; 400mg), ground lactose (3420mg) and light anhydrous silicic acid (180mg) were mixed in the small grinder for one minute to yield an NF-κB decoy mixture powder (2.5μg/mg) .

NF-κB decoy mixture powder (2.5μg/mg; 400mg), ground lactose (3420mg) and light anhydrous silicic acid (180mg) were mixed in the small grinder for one minute to yield an NF-κB decoy mixture powder (0.25μg/mg) .

NF-κB decoy mixture powder (0.25μg/mg; 800mg), ground lactose (3040mg) and light anhydrous silicic acid (160mg) were mixed in the small grinder for one minute to yield an NF-κB decoy mixture powder (0.05μg/mg) .

(Experimental procedures)

Male BN rats were given antigen ovalbumin OA (1mg/rat) into the femoral muscle and Bordetella pertusis (1.25 x $10^{10}$/rat) in peritoneal cavity and actively sensitized. Twelve or thirteen days after the sensitization, 0.5 % antigen in saline was inhaled under anesthesia, and raised allergic response. Three days after the antigen inhalation, airway responsiveness against acetylcholine (ACh) was studied. The airway responsiveness against ACh was assessed by inhalations across two-minute intervals. Each inhalation was performed for 10 seconds at a concentration which was sequentially doubled each time, starting from 0.1 mg/mL. The concentration of ACh which increased 5cmH$_2$O of the airway pressure from ACh inhalation initiation was calculate as Provocation Concentration 5cmH$_2$O (PC$_{5cmH20}$ ACh) ACh (mg/mL). After determination of airway responsiveness, an airway wash was performed, and the number of cells infiltrated to the airway was calculated. As negative control, a group in which sensitized rats inhaled saline, and a control group in which sensitized rats inhaled an antigen with a solvent, were prepared.

Further, four grams of NF-κB decoy mixture powder (0.05, 0.25, 1.25, and 6.25 μg/mg) prepared by the afore-said method were inhaled into rats under anesthesia three days prior to the antigen inhalation. The average body weight of BN rat used in the present example was 200 g and thus the dosage amount of NF-κB decoy was 1μg/kg, 5μg/kg, 25μg/kg and 125μg/kg, respectively.

(Experimental results)

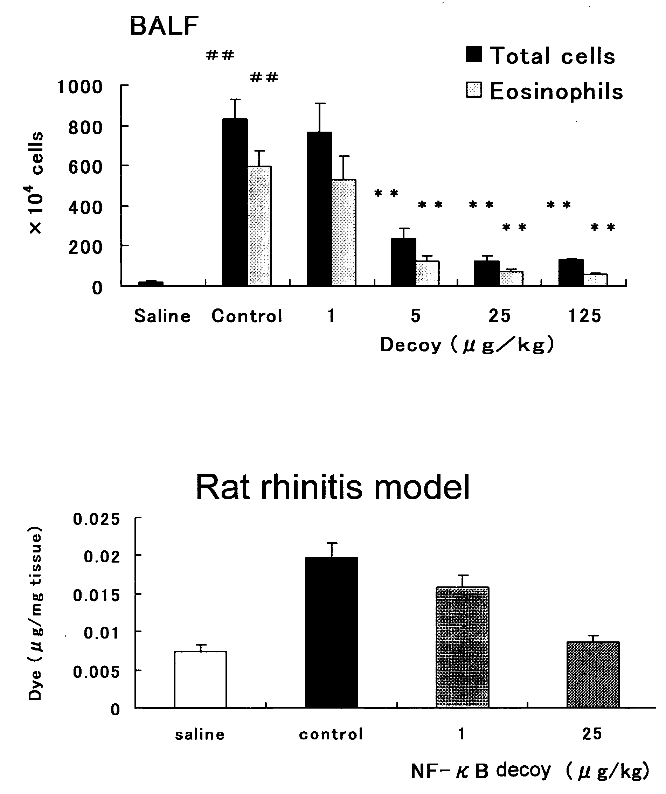

As shown in Figures **7** and **8** (Figures **7** and **8**: effects of NF-κB decoy in a rat asthma model. The legend of the figure is as follows: ##: $p<0.01$ vs saline group (Student's t-test);*, **: $p<0.05$, 0.01, respectively, vs control group (Dunnett's multiple comparison test)), compared to saline control group, it was observed that in the control group, there was an evident enhancement of airway responsiveness (airway hypersensitivity) and infiltration of inflammatory cells mainly consisting of eosinophils. NF-κB decoy suppresses these changes in a dosage dependent manner starting from 5μg/kg, and reaches maximum suppression at 25 and 125 μg/kg. This clarified the efficacy of NF-κB decoy powder inhalation agent in a rat asthma model.

TABLE 2 rat asthma model appendix

| | log (PC50ACh) | ACh (mg/mL) | Total cells | Eosinophils |
|---|---|---|---|---|
| **Saline** | | | $(*10^4)$ | $(*10^4)$ |
| | 0.576 | 3.769 | 12.0 | 0.0 |
| | 0.565 | 3.676 | 16.5 | 1.5 |
| | 0.019 | 1.044 | 36.0 | 6.0 |
| | 0.269 | 1.856 | 12.0 | 0.0 |
| | 0.666 | 4.631 | 19.5 | 1.5 |
| | 0.258 | 1.811 | 25.5 | 0.0 |
| mean | 0.392 | 2.467 | 20.3 | 1.5 |
| SE | 0.102 | | 3.8 | 0.9 |

| **Control** | | | | |
|---|---|---|---|---|
| | -0.869 | 0.135 | 1026.0 | 789.0 |
| | -0.454 | 0.352 | 663.0 | 501.0 |
| | -0.770 | 0.170 | 756.0 | 576.0 |
| | -0.866 | 0.136 | 567.0 | 399.0 |
| | -0.153 | 0.704 | 723.0 | 402.0 |
| | -0.264 | 0.544 | 1326.0 | 945.0 |
| | -0.479 | 0.332 | 762.0 | 555.0 |
| mean | -0.551 | 0.281 | 831.9 | 595.3 |
| SE | 0.110 | | 98.0 | 76.6 |

| **decoy 1μg/kg** | | | | |
|---|---|---|---|---|
| | -0.080 | 0.832 | 336.0 | 210.0 |
| | 0.072 | 1.181 | 1182.0 | 894.0 |
| | -0.398 | 0.400 | 1032.0 | 768.0 |
| | -0.668 | 0.215 | 306.0 | 180.0 |
| | -0.065 | 0.861 | 516.0 | 321.0 |
| | -0.247 | 0.566 | 822.0 | 549.0 |
| | -0.473 | 0.336 | 1155.0 | 801.0 |
| mean | -0.266 | 0.543 | 764.1 | 531.9 |

Table continued

| decoy 1μg/kg | | | | |
|---|---|---|---|---|
| SE | 0.099 | | 142.9 | 112.5 |

| decoy 5μg/kg | | | | |
|---|---|---|---|---|
| | -0.319 | 0.479 | 429.0 | 189.0 |
| | -0.143 | 0.720 | 327.0 | 198.0 |
| | 0.010 | 1.023 | 204.0 | 117.0 |
| | 0.028 | 1.066 | 111.0 | 48.0 |
| | 0.084 | 1.213 | 111.0 | 57.0 |
| | -0.283 | 0.522 | 336.0 | 198.0 |
| | 0.320 | 2.091 | 141.0 | 78.0 |
| mean | -0.043 | 0.905 | 237.0 | 126.4 |
| SE | 0.084 | | 48.0 | 25.6 |

| decoy 25μg/kg | | | | |
|---|---|---|---|---|
| | 0.204 | 1.600 | 105.0 | 63.0 |
| | 0.311 | 2.046 | 78.0 | 36.0 |
| | 0.218 | 1.654 | 234.0 | 120.0 |
| | 0.309 | 2.038 | 207.0 | 102.0 |
| | -0.059 | 0.874 | 156.0 | 78.0 |
| | 0.050 | 1.123 | 147.0 | 66.0 |
| | 0.122 | 1.324 | 123.0 | 60.0 |
| mean | 0.165 | 1.463 | 150.0 | 75.0 |
| SE | 0.052 | | 20.9 | 10.6 |

| decoy 125μg/kg | | | | |
|---|---|---|---|---|
| | 0.191 | 1.553 | 93.0 | 39.0 |
| | 0.307 | 2.027 | 99.0 | 51.0 |
| | 0.045 | 1.109 | 123.0 | 72.0 |
| | 0.505 | 3.200 | 105.0 | 54.0 |
| | 0.394 | 2.479 | 141.0 | 57.0 |
| | -0.034 | 0.925 | 159.0 | 57.0 |
| | -0.016 | 0.964 | 174.0 | 84.0 |
| mean | 0.199 | 1.581 | 127.7 | 59.1 |
| SE | 0.080 | | 11.8 | 5.5 |

(Example 5: Effects of NF-κB decoy on an allergic rhinitis model)

The present example demonstrates the effects of NF-κB decoy on a rat rhinitis model.

(Experimental methodology)

Antigen 2,4-dinitrophenyl ovalbumin (DNP-OA) (1 mg/rat) and Bordetella pertussis (1.25 x $10^{10}$) were given to a male SD rat on the footpads of four limbs subcutaneously for active sensitization. Nine days after sensitization, antigens were administered to the nose on both sides (10% DNP-OA $\times$ 0.02 mL/site $\times$ 2 site), and thereby caused allergic response. Six hours after the onset of rhinitis, a stain (Pontamin Sky-Blue) was i.v. administered, and the subject was dissected nine hours after the treatment, when the peak was observed. Tissues around the nasal septum were removed and the level of leakage of the stain was measured. A negative control group in which sensitized rats were given saline in the nose, and a control group in which sensitized rats were given an antigen in the nose and a solvent, were prepared. NF-κB decoy was dissolved in saline at a concentration of 5 and 125 μg/mL, and 20 μL each was dropped into the nose per each nostril, so that the dosage amounts were 0.2 μg and 5 μg, respectively, three days prior thereto. The average body weight of SD rat used in the present example was 200 g, and thus the dosage amount of NF-κB decoy was 1μg/kg and 25μg/kg, respectively.

(Experimental Results)

The legend of the figure 9 is as follows: ##: p<0.01 vs saline group (Student's t-test);*, ** : p<0.05,0.01, respectively, vs control group (Dunnett's multiple comparison test. As shown in Figure **9**, which depicts the effects of NF-κB decoy in a rat rhinitis model, compared to the negative control (saline) group, positive control group was observed to have evident enhancement of blood permeability, and thus it was possible to confirm that the symptoms of rhinitis were presented. On the other hand, NF-κB suppressed the enhancement of the blood permeability in a volume-dependent manner starting from 1 μg/kg, and showed substantially complete suppression of the enhancement of the blood permeability at 25 μg/kg. As such, the efficacy of NF-κB decoy in a rat rhinitis model was demonstrated.

TABLE 3 Rhinitis Appendix

|                | saline | control | NF-kB 1 | NF-kB 25 |
|----------------|--------|---------|---------|----------|
|                | 0.0052 | 0.0193  | 0.0196  | 0.0082   |
|                | 0.0067 | 0.0217  | 0.0164  | 0.0091   |
|                | 0.0068 | 0.0184  | 0.0152  | 0.0086   |
|                | 0.0105 | 0.0165  | 0.0123  | 0.0064   |
|                |        | 0.0183  | 0.0115  | 0.0099   |
| average        | 0.0073 | 0.0188  | 0.0150  | 0.0084   |
| standard error | 0.0011 | 0.0010  | 0.0013  | 0.0005   |

(Example 6: Preparation of a beagle dog COPD model)

Beagle dogs (body weight 9-14 kg) were anesthetized by subcutaneous injection of an anesthetic for animal (Selactal (R) (Bayer)). A bronchoscope is inserted into the trachea by means of endotracheal tube. Porcine pancreas elastase (50 mg; 3750 units, available from Nacalai Tesque) was dissolved in 5 ml of saline, and atomization catheter (available from Olympus or the like) was introduced to the peripheral trachea and dispersed to the global right lung from five to ten times. Those which passed about one month after the dispersion was used as the COPD model lung. The left lung was used as the normal lung for comparison.

(Example 7: Administration of NF-κB decoy into a beagle dog model)

A microparticle powder having particle size of 3μ m or less was produced by grinding NF-κB decoy according to a method described in Example 4.
NF-κB (200 μg) is dispersed into COPD model lung as a NF-κB decoy mixed powder in a diffused manner in the global right lung by means of bronchoscope for five to ten times. In order to observe the changes between before and after the therapy of NF-κB decoy, blood flow is measured by MRI and compared.

(Comparison results in MRI)

MRI was used for preparing photographs in a supine position after systemic anesthesia of the beagle dog. A vein route was confirmed in the right arteria carotis interna and conventionally available contrast agent was injected, and about three seconds thereafter, intrapulmonary blood vessels are contrasted. Due to the contrast effects, MRI signal intensity had been enhanced. MRI signal intensity depends on the blood flow, thus more the blood flows, the more the signal intensifies. Due to individual differences amongst the blood volume of pulmonary artery, in order to diminish such individual differences, MRI signal values of right lung which has received administration was divided by that of the normal left lung, were used for comparison between the left and the right. After the injection of 3 ml of conventional contrast agent, imaging was initiated. For about 120 seconds after the injection of the contrast agent, one hundred circular cross sections were photographed and signal values of the section limited to the lung parenchyma are consecutively measured, and analysed. The left-right ratio of the MRI intrapulmonary blood volumes was also calculated. Based on these results, the ratio of the left and right signal values were measured, it is observed that there was no difference between the left and the right lungs in the normal dog, whereas in the COPD model, right lung blood flow was reduced, and the NF-κB decoy therapy model is improved with respect to blood flow, and approximates the normal condition. That is, it is recognized that administration of NF-κB decoy provided significant improvement of blood flow.

Although certain preferred embodiments have been described herein, it is not intended that such embodiments be construed as limitations of the scope of the invention except as set forth in the appended claims. Various other modifications and equivalents of this invention will be apparent to, and can be readily made by, those skilled in the art, after reading the description herein with specific and preferable embodiments of the present invention in view of the common general knowledge of the art. All patents, published patent applications and publications cited herein are incorporated by reference as if set forth fully herein.

INDUSTRIAL APPLICABILITY

A pharmaceutical composition or a carrier used for treating a disease related to a disease caused by the expression of a gene controlled by NF-κB or abnormality of eosinophils. Topical administration of the pharmaceutical composition according to the present invention is non-invasive and can be repetitively provided.

**Claims**

1. A pharmaceutical composition for treating and/or preventing a disease, disorder and/or condition of the respiratory system due to expression of a gene regulated by NF-κB, comprising an NF-κB decoy and a pharmaceutically acceptable carrier.

2. A composition according to Claim 1, wherein said NF-κB decoy is a NF-κB decoy or a derivative, variant or fragment thereof, and the derivative, variant or fragment has a biological activity.

3. A composition according to Claim 1, wherein said NF-KB decoy is a decoy set forth in SEQ. ID NO: 1.

4. A composition according to Claim 1 wherein said disease, disorder and/or condition of respiratory system is airway inflammatory disease, airway stenosis or nasal cavity inflammatory disease.

5. A composition according to Claim 1, wherein said disease, disorder and/or condition of the respiratory system is COPD, asthma or rhinitis.

6. A composition according to Claim 1, wherein said disease, disorder and/or condition of the respiratory system is COPD.

7. A composition according to Claim 1, wherein said disease, disorder and/or condition of the respiratory system is asthma.

8. A composition according to Claim 1, wherein said disease, disorder and/or condition of the respiratory system is rhinitis.

9. A composition according to Claim 1, wherein said pharmaceutically acceptable carrier is a hydrophilic polymer, a carbohydrate or an insoluble additive.

10. A composition according to Claim 1, wherein said pharmaceutically acceptable carrier is at least one type selected from the group consisting of a liposome, lactose, trehalose, sucrose, mannitol and xylitol.

11. A composition for treating and/or preventing a disease, disorder and/or condition of the respiratory system relating to an eosinophil abnormality, comprising NF-κB decoy and a pharmaceutically acceptable carrier.

12. A composition according to Claim 11, wherein said NF-κB decoy is a NF-κB decoy or a derivative, variant or fragment thereof, and the derivative, variant or fragment has a biological activity.

13. A composition according to Claim 11, wherein said NF-κB decoy is a decoy set forth in SEQ. ID NO: 1.

14. A composition according to Claim 11 wherein said disease, disorder and/or condition of the respiratory system is airway inflammatory, airway stenosis or nasal cavity inflammatory disease, disorder and/or condition.

15. A composition according to Claim 11, wherein said disease, disorder and/or condition of the respiratory system is COPD, asthma or rhinitis.

16. A composition according to Claim 11, wherein said disease, disorder and/or condition of the respiratory system is an asthma selected from the group consisting of bronchial asthma, infantile asthma, allergic asthma, atopic asthma, steroid refractory asthma, non-allergic asthma, endogenous asthma, exogenous asthma, aspirin asthma, cardiac asthma and infectious asthma; or a rhinitis selected from the group consisting of allergic rhinitis, pollinosis, acute rhinitis, chronic rhinitis, hypertrophic rhinitis, chronic sinusitis (emphysema) and deflected septum.

17. A composition according to Claim 11, wherein said pharmaceutically acceptable carrier is a hydrophilic polymer or a carbohydrate.

18. A composition according to Claim 11, wherein said pharmaceutically acceptable carrier is one or more selected from the group consisting of a liposome, lactose, trehalose, sucrose, mannitol and xylitol.

**19.** A dosage formulation for respiratory system adapted for administration to the respiratory system, comprising a composition according to any one of Claims 1-18.

**20.** A dosage formulation according to Claim 19, wherein said administration to the respiratory system comprises administration into the airway or transairway absorption.

**21.** A dosage formulation according to Claim 19, wherein said administration to the respiratory system is administration to the airway by atomization or inspiration.

**22.** A dosage formulation according to Claim 19, wherein said administration into the airway comprises administration by metered dose inhaler (MDI), dry powder inhaler (DPI) or nebulizer.

**23.** A dosage formulation according to Claim 19, wherein said composition is provided as a dry powder.

**24.** A dosage formulation according to Claim 23, wherein said dry powder is a microparticle produced by means of one of selected from the group consisting of a bowl mill, a bead mill, a jet mill, an ultimizer, a mortar, a stonemill, spray drying and supercritical fluid.

**25.** A dosage formulation according to Claim 23, wherein the dry powder has an aerodynamic average particle size of about 0.01 to about 50 micrometer.

**26.** A dosage formulation according to Claim 23, wherein the dry powder has an aerodynamic average particle size of about 0.05 to about 30 micrometer.

**27.** A dosage formulation according to Claim 23, wherein the dry powder has an aerodynamic average particle size of about 0.1 to about 10 micrometer.

**28.** A dosage formulation according to Claim 19, wherein a dosage of 10 $\mu$g to 100 mg per round is provided.

**29.** A dosage formulation according to Claim 19, wherein a dosage of 50 $\mu$g to 50 mg per round is provided.

**30.** A dosage formulation according to Claim 19, wherein a dosage of 10 mg or less per round is provided.

**31.** A dosage formulation according to Claim 19, wherein the administration to the respiratory system comprises nasal absorption.

**32.** A dosage formulation according to Claim 31, which is a formulation selected from the group consisting of a nasal drop, a nasal spray agent, an agent for nebulizer, an agent for a respirator and powder administration formulation.

**33.** A dosage formulation according to Claim 31, which is a nasal drop for rhinitis.

**34.** A dosage formulation according to Claim 19, wherein the NF-κB decoy is encapsulated in an HVJ-E envelope vector.

**35.** A dosage formulation according to Claim 19 wherein the administration to the respiratory system comprises administration to the lung.

**36.** A device for treating the respiratory system comprising a composition according to any one of Claims 1-18, and means for administering the composition to the respiratory system.

**37.** A device according to Claim 36, wherein the means for administration to the respiratory system comprises means selected from the group consisting of means for administration to the lung, means for transairway administration, means for transairway absorption and means for nasal absorption.

**38.** A device according to Claim 36, wherein said means for administration to the airway comprises a metered dose inhaler (MDI), dry powder inhaler (DPI) or a nebulizer.

**39.** A method for manufacturing a particle comprising NF-κB decoy, comprising the steps of:

A) providing the decoy and a pharmaceutically acceptable carrier;

B) drying and heating the decoy and the carrier to a temperature sufficient for particulization; and

C) obtaining particles having a desired particle size.

40. A method according to Claim 39 wherein the temperature is 50 degree Celcius or lower.

41. A method according to Claim 39 wherein the temperature is 100 degree Celcius or lower.

42. A method according to Claim 39, wherein the dry powder has an aerodynamic average particle size of about 0.01 to about 50 micrometer.

43. A method according to Claim 39, wherein the dry powder has an aerodynamic average particle size of about 0.05 to about 30 micrometer.

44. A method according to Claim 39, wherein the dry powder has an aerodynamic average particle size of about 0.1 to about 10 micrometer.

45. A method for treating and/or preventing a disease, disorder and/or condition of the respiratory system due to expression of a gene regulated by NF-κB, comprising the step of:

A) administration of a composition comprising an NF-κB decoy and a pharmaceutically acceptable carrier to the respiratory system of a subject.

46. A method according to Claim 45, wherein said disease, disorder and/or condition of the respiratory system is airway inflammatory disease, airway stenosis or nasal cavity inflammatory disease.

47. A method according to Claim 45, wherein said disease, disorder and/or condition of respiratory system is COPD, asthma or rhinitis.

48. A method according to Claim 45, wherein said administration to the respiratory system comprises administration into the airway or transairway absorption.

49. A method according to Claim 45, wherein said administration to the respiratory system is administration to the airway by atomization or inspiration.

50. A method according to Claim 45, wherein said administration into the airway comprises administration by metered dose inhaler (MDI), dry powder inhaler (DPI) or nebulizer.

51. A method according to Claim 45, wherein administration is achieved by means selected from the group consisting of a nasal drop, a nasal spray, a nebulizer, a respirator and powder administration.

52. A method for treating and/or preventing a disease, disorder and/or condition of the respiratory system due to an eosinophil abnormality, comprising the step of:

A) administration of a composition comprising an NF-κB decoy and a pharmaceutically acceptable carrier to the respiratory system of a subject.

53. Use of an NF-κB decoy for the manufacture of a medicament for treating and/or preventing a disease, disorder and/or condition of the respiratory system due to expression of a gene regulated by NF-κB.

54. Use of an NF-κB decoy for the manufacture of a medicament for treating and/or preventing a disease, disorder and/or condition of respiratory system due to an eosinophil abnormality.

FIG.1A

**Method**

OVA
subcutaneous
injection
                Introduction    OVA
                of decoy     challenge   sacrifice

Day0                     Day12          Day14 ⟶ 8,24hr
after OVA
challenge

OVA
subcutaneous
injection
                Introduction    OVA
                of decoy     challenge   sacrifice

Day0                   Day12 &13   Day14 ⟶ 24hr after
OVA
challenge

FIG.1B

Inhalation of OVA and decoy into rat

nasal inhalation

aerosol

Decoy :2mg/4ml
OVA :5%wt. × 5min
O2 flow 7-8 ℓ/min

**Inhalation of decoy into rat (2)**

FIG.1C

Insertion of needle

Decoy :2 or 4mg/20ml x 2
Air flow 7-8 ℓ/min, 60min
normal inhalation

## FIG.2A

cell population

cell count

EP 1 642 591 A1

FIG.2B

# BAL cell count

EP 1 642 591 A1

# BAL Eosinophil %

FIG.2C

# FIG.3A    **BAL Eosinophil cell count**

**cell count**

**x10E6**

naked: naked decoy 500ug

HVJ(200): HVJ-E 10000HAU & decoy 200ug

HVJ(500): HVJ-E 25000HAU & decoy 500ug

Vector: HVJ-E 25000HAU without decoy

#: vs. OVA

**FIG.3B**      **BAL Eosinophil %**

naked: naked decoy 500ug

HVJ(200): HVJ-E 10000HAU & decoy 200ug      #: vs. OVA

HVJ(500): HVJ-E 25000HAU & decoy 500ug

Vector: HVJ-E 25000HAU without decoy

FIG. 4

NF-KB DECOY
BALL MILL 10HRS
20031204-1

120504 20KV X1.00K 24.0um

## AHR

log(PC$_{5cmH2O}$ACh)

Decoy (μg／kg)

Saline  Control  1  5  25  125

## BALF

## Rat rhinitis model

**INTERNATIONAL SEARCH REPORT**

| International application No. |
|---|
| PCT/JP2004/009838 |

A. CLASSIFICATION OF SUBJECT MATTER
Int.Cl⁷ A61K45/00, 48/00, 31/7088, 9/08, 9/12, 9/127, 9/14, 9/72, 47/10, 47/26, 47/30, 35/76, A61P11/00, 11/02, 11/06

According to International Patent Classification (IPC) or to both national classification and IPC

B. FIELDS SEARCHED

Minimum documentation searched (classification system followed by classification symbols)
Int.Cl⁷ A61K45/00, 48/00, 31/7088, 9/08, 9/12, 9/127, 9/14, 9/72, 47/10, 47/26, 47/30, 35/76, A61P11/00, 11/02, 11/06

Documentation searched other than minimum documentation to the extent that such documents are included in the fields searched

Electronic data base consulted during the international search (name of data base and, where practicable, search terms used)
MEDLINE(STN), BIOSIS(STN), CAPLUS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X Y | WO 96/35430 A1 (Fujisawa Pharmaceutical Co., Ltd.), 14 November, 1996 (14.11.96), Full text; particularly, Claims; example 1; page 5, lines 7 to 19 & EP 824918 A1 & JP 3474879 B & US 6262033 B1 | 1-18,53,54 19-44 |
| X Y | JP 2002-193813 A (AnGes MG, Inc.), 10 July, 2002 (10.07.02), Full text; particularly, Claims; Par. Nos. [0033], [0048], [0049], [0062] (Family: none) | 1-5,7,9-18, 53,54 19-44 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 05 October, 2004 (05.10.04) | Date of mailing of the international search report 02 November, 2004 (02.11.04) |
|---|---|
| Name and mailing address of the ISA/ Japanese Patent Office | Authorized officer |
| Facsimile No. | Telephone No. |

Form PCT/ISA/210 (second sheet) (January 2004)

EP 1 642 591 A1

**INTERNATIONAL SEARCH REPORT**

International application No.

PCT/JP2004/009838

C (Continuation). DOCUMENTS CONSIDERED TO BE RELEVANT

| Category* | Citation of document, with indication, where appropriate, of the relevant passages | Relevant to claim No. |
|---|---|---|
| X | LEE, Hern-Ku et al., Inhibition of murine asthmatic reactions by antisense | 1-5,7,9-18, 53,54 |
| Y | oligonucleotide to p65 subunit of nuclear factor (NF)-κB, FASEB Journal, 2001, Vol.15, No.4, page A663, 523.9 | 19-44 |
| X | SUGIURA, Hisatoshi et al., New therapies for the airway inflammation in COPD, Lung | 1-6,9-15,17, 18,53,54 |
| Y | Perspectives, 2002, Vol.10, No.2, pages 174 to 178 | 19-44 |
| Y | US 6303582 B1 (INHALE THERAPEUTIC SYSTEMS, INC.), 16 October, 2001 (16.10.01), Full text & US 5994314 A | 19-44 |
| Y | JP 2001-526634 A (Massachusetts Institute of Technology), 18 December, 2001 (18.12.01), Full text & WO 98/31346 A1    & EP 954282 A1 & US 5855913 A | 19-44 |

Form PCT/ISA/210 (continuation of second sheet) (January 2004)

41

**INTERNATIONAL SEARCH REPORT**

| International application No. |
| --- |
| PCT/JP2004/009838 |

**Box No. I    Nucleotide and/or amino acid sequence(s) (Continuation of item1.b of the first sheet)**

1.  With regard to any nucleotide and/or amino acid sequence disclosed in the international application and necessary to the claimed invention, the international search was carried out on the basis of:

    a.   type of material

         [X]  a sequence listing

         [ ]  table(s) related to the sequence listing

    b.   format of material

         [ ]  in written format

         [X]  in computer readable form

    c.   time of filing/furnishing

         [X]  contained in the international application as filed

         [ ]  filed together with the international application in computer readable form

         [ ]  furnished subsequently to this Authority for the purposes of search

2.  [ ]  In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3.  Additional comments:

Form PCT/ISA/210 (continuation of first sheet (1)) (January 2004)

**INTERNATIONAL SEARCH REPORT**

| International application No. |
|---|
| PCT/JP2004/009838 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 45-52
   because they relate to subject matter not required to be searched by this Authority, namely:
   Claims 45 to 52 pertain to methods for treatment of the human body by surgery or therapy and thus relate to a subject matter which this International Searching Authority is not required to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
   The special technical feature in claims 1 to 38, 53 and 54 relates to using an NF-κB decoy in treating and/or preventing respiratory diseases, while the special technical feature of claims 39 to 44 relates to a process for producing particles containing an NF-κB decoy.
   Since there is no technical relationship between these groups of inventions involving one or more of the same or corresponding special technical features, these groups of inventions are not considered as being so linked as to form a single general inventive concept.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**          ☐ The additional search fees were accompanied by the applicant's protest.

                              ☐ No protest accompanied the payment of additional search fees.

Form PCT/ISA/210 (continuation of first sheet (2)) (January 2004)